# EUROPEAN PATENT APPLICATION

(11) **EP 3 610 874 A1**
(43) Date of publication of application: **19.02.2020**
(21) Application number: 18783681.2
(22) Date of filing: 13.04.2018
(51) Int. Cl.: A61K 31/443, A61K 31/4433, A61K 31/4436, A61K 31/444, A61K 31/45, A61P 25/04

(54) **PHARMACEUTICAL COMPOSITION CONTAINING MOR AGONIST AND KOR AGONIST, AND USES THEREOF**

(30) Priority: 14.04.2017 CN 201710242435
(71) Applicant: Jiangsu Hengrui Medicine Co., Ltd., Lianyungang, Jiangsu 222047 (CN)
(72) Inventor: NIU, Zhuolu, Lianyungang Jiangsu 222047 (CN); CAO, Guoqing, Lianyungang Jiangsu 222047 (CN); YANG, Changyong, Lianyungang Jiangsu 222047 (CN); ZHANG, Lianshan, Lianyungang Jiangsu 222047 (CN)
(74) Representative: Potter Clarkson
(86) International application number: PCT/CN2018/082912
(87) International publication number: WO 2018/188641

(57) **Abstract**

Provided are uses of the combination of a µ opioid receptor (MOR) agonist and a κ opioid receptor (KOR) agonist in the preparation of drugs for relieving and/or treating pain. The MOR agonist is selected from the compound represented in formula (I), or a tautomer, mesomer, racemate, enantiomer or diastereomer thereof, or a mixture thereof, or a pharmaceutically acceptable salt thereof. Also provided is a pharmaceutical composition containing the MOR agonist and the KOR agonist.

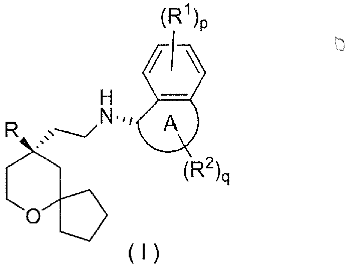

## Description

### FIELD OF THE INVENTION

The present invention belongs to the field of medicine, and relates to a use of a combination of a MOR agonist and a KOR agonist in the preparation of a medicament for alleviating and/or treating pain.

### BACKGROUND OF THE INVENTION

Pain refers to a person's functional or substantial feelings. The classification of pain is complicated. According to the etiology, the pain is mainly classified into traumatic pain, pathological pain, pain caused by metabolic diseases, neuropathic pain, pain caused by tissue and organ malformation, psychological pain, and pain caused by combined factors. According to the course, the pain is mainly classified into transient pain, acute pain and chronic pain. According to the degree, the pain is classified into faint pain, mild pain, serious pain and severe pain. According to the anatomy, the pain is mainly classified into headache, maxillofacial pain, cervical occipital pain, neck and shoulder pain, upper limb pain, chest pain, abdominal pain, lumbocrural pain. According to the location and cause, the pain is classified into peripheral pain, central pain and psychological pain. The causes of pain diseases are complicated, and the symptoms are different. The degree of tolerance to pain and the response to treatment vary greatly between patients. At present, the clinically used drugs for treating pain mainly include anti-inflammatory analgesics, narcotic analgesics, local anesthetics, anti-epileptic drugs, anti-depressants and the like. Although there are many drugs for analgesia, there are still problems such as constipation, respiratory depression, sedation and lethargy, nausea and vomiting, acute poisoning, physical dependence and drug resistance, psychological dependence and the like.

Opioids are commonly used analgesics in clinical practice, and especially play an important role in treating patients with severe pain and advanced cancer. Opioids produce an analgesic effect primarily through acting on the opioid receptor. The opioid receptor is a member of the G protein-coupled receptor superfamily, and participates in a variety of physiological activities such as analgesia, inhibition of gastrointestinal motility, respiratory depression, myocardial protection, immune response and the like. In general, the opioid receptor can be divided into four subtypes: µ opioid receptor (MOR), δ opioid receptor (DOR), κ opioid receptor (KOR), and opioid receptor like-1 (ORL-1). Studies find that MOR receptor has the strongest binding ability to morphin-1. Therefore, the opioid analgesics used in clinical practice are MOR agonists, such as morphine, tramadol, fentanyl, oxycodone and the like. However, long-term use of these drugs can cause severe side effects such as analgesia tolerance, dependence, addiction and the like. The MOR agonist currently in phase III clinical study includes TRV-130, which is developed by Trevena Inc. WO2017063509 (PCT patent application No. PCT/CN2016/101064) discloses a novel MOR agonist, whose structure is shown as follows:

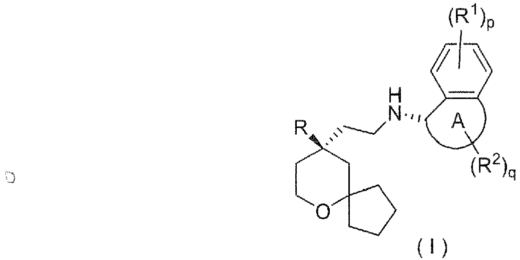

WO2012129495 discloses a MOR agonist with a similar structure.

The study of the structure and function of various subtypes of opioid receptors breaks the previous understanding that a highly selective ligand targeting a single opioid receptor will have a high activity and low toxicity side effect. At present, more studies find that a highly selective agonist can enhance side effects instead of reducing them. Studies find that there are different degrees of structural or functional interactions between different subtypes of opioid receptors, which participate together in physiological activities such as analgesia and the like. The study by Fujita-Hamabe et al. (Journal of Pharmacy and Pharmacology, 2010, 62(8): 995-1002) demonstrates that KOR can inhibit the desensitization of MOR, accelerate the intracellular circulation of MOR to increase surface receptor, and reduce the activity of protein kinase C, thereby inhibiting the analgesic tolerance and dependence of MOR agonists. The study by Cunha TM et al. ([J]. Molecular pain, 2012, 8(1): 10) finds that the activation of peripheral MOR can inhibit inflammatory pain and prostatin E2-induced progressive hyperalgesia. It has been reported that the activation of KOR can also inhibit inflammatory hyperalgesia, and its mechanism may involve the activation of PI3K γ/AKT signaling pathway through nNOS/NO signaling pathway. Rong LIU et al. ([J]. China Pharmaceuticals, 2016, 25(22): 41-44) report the analgesia and sedation effect of the KOR agonist nalbuphine and MOR agonist sufentanil after Elderly total hip arthroplasty, demonstrating that the combined administration has a better analgesic effect than single administration, and significantly reduces adverse reactions such as nausea, vomiting, skin itch and the like. Nalfurafine (approved in Japan on May 2015) has been approved as a KOR agonist.

CN107098871A (Chinese patent application No. 201710095021.X) discloses a novel KOR agonist, whose structure is shown as follows:

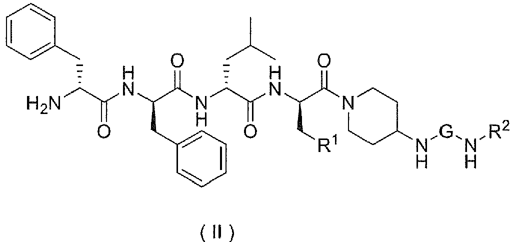

WO2008060552 discloses a KOR agonist with a similar structure, and its use for analgesia in combination with other opioid receptor agonists, NSAIDs and anti-depressants. WO2016073443 discloses a use of a similar KOR agonist for treating surgical pain and sclerous tissue pain. WO2008057608 discloses a use of a combination of a similar KOR agonist and a MOR agonist for treating pain as well as the decrease of the dose of MOR agonist and adverse reactions. WO2015065867 discloses a use of a similar KOR agonist administrated after administration of a MOR agonist for reducing the vomiting induced by the MOR agonist. In summary, the combined administration of a MOR agonist and a KOR agonist is a potential method for alleviating and/or treating pain. The present invention provides a use of a combination of a novel structure of MOR agonist and a KOR agonist in the preparation of a medicament for alleviating and/or treating pain.

### SUMMARY OF THE INVENTION

The technical problem to be solved by the present invention is to provide a use of a combination of a MOR agonist and a KOR agonist in the preparation of a medicament for alleviating and/or treating pain.

The MOR agonist is selected from a compound of formula (I) having the follwing structure: 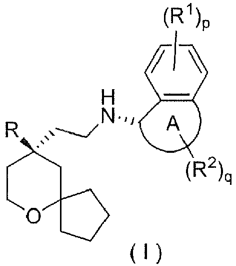 or a tautomer, mesomer, racemate, enantiomer, diastereomer thereof, or mixture thereof, or a pharmaceutically acceptable salt thereof,
wherein:
ring A is selected from the group consisting of cycloalkyl and heterocyclyl;
R is selected from the group consisting of aryl and heteroaryl, wherein the aryl and heteroaryl are each optionally substituted by one or more substituents selected from the group consisting of alkyl, haloalkyl, halogen, amino, nitro, cyano, alkoxy, haloalkoxy, hydroxyalkyl, cycloalkyl, heterocyclyl, aryl, heteroaryl, -OR³, -C(O)R³, -C(O)OR³, -S(O)ₘR³ and -NR⁴R⁵;
each R¹ is identical or different and each is independently selected from the group consisting of hydrogen, alkyl, alkoxy, haloalkyl, halogen, amino, nitro, hydroxy, cyano, cycloalkyl, heterocyclyl, aryl, heteroaryl, -OR³, -C(O)R³, -C(O)OR³, -S(O)ₘR³ and -NR⁴R⁵, wherein the alkyl, haloalkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each R² is identical or different and each is independently selected from the group consisting of hydrogen, alkyl, alkoxy, haloalkyl, halogen, amino, nitro, hydroxy, cyano, oxo, alkenyl, cycloalkyl, heterocyclyl, aryl, heteroaryl, -OR³, -C(O)R³, -C(O)OR³, -S(O)ₘR³ and -NR⁴R⁵, wherein the alkyl, alkoxy, alkenyl, haloalkyl, cycloalkyl, heterocyclyl, aryl and heteroaryl are each optionally substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl and heteroaryl;
or two R² are taken together to form a cycloalkyl or heterocyclyl, wherein the cycloalkyl or heterocyclyl is optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R³ is selected from the group consisting of hydrogen, alkyl, deuterated alkyl, amino, alkoxy, hydroxyalkyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, halogen, hydroxy, amino, nitro, cyano, alkoxy, hydroxyalkyl, cycloalkyl, heterocyclyl, aryl and heteroaryl;
R⁴ and R⁵ are each independently selected from the group consisting of hydrogen, alkyl, alkoxy, hydroxyalkyl, hydroxy, amino, alkoxycarbonyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
p and q are each independently 0, 1, 2, 3 or 4; and
m is 0, 1 or 2.

In an embodiment of the present invention, the MOR agonist is selected from a compound of formula (I-A): 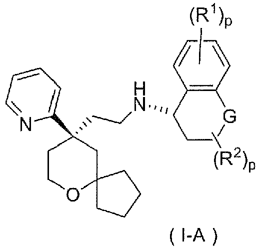 or a tautomer, mesomer, racemate, enantiomer, diastereomer thereof, or mixture thereof, or a pharmaceutically acceptable salt thereof,
wherein:
G is selected from the group consisting of a bond, CR^{a}R^{b}, C=O, NR⁴ and oxygen;
R^{a} and R^{b} are each independently selected from the group consisting of hydrogen, alkyl, alkoxy, haloalkyl, halogen, amino, nitro, hydroxy, cyano, cycloalkyl, heterocyclyl, aryl, heteroaryl, -OR³, -C(O)R³, -C(O)OR³, -S(O)ₘR³ and -NR⁴R⁵, wherein the alkyl, haloalkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
or R^{a} and R^{b} are taken together to form a cycloalkyl or heterocyclyl, wherein the cycloalkyl or heterocyclyl is optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl; and
R¹-R⁵, p, m and q are as defined in formula (I).

In another embodiment of the present invention, the MOR agonist is selected from a compound of formula (I-B): 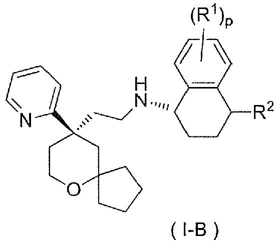 or a tautomer, mesomer, racemate, enantiomer, diastereomer thereof, or mixture thereof, or a pharmaceutically acceptable salt thereof,
wherein:
R¹, R² and p are as defined in formula (I).

In an embodiment of the present invention, the MOR agonist is selected from the group consisting of: 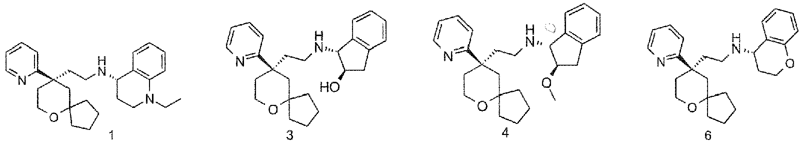 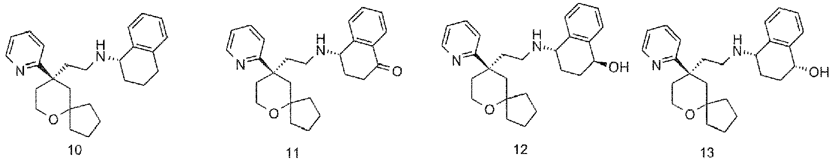 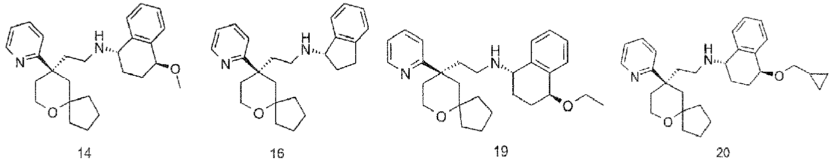 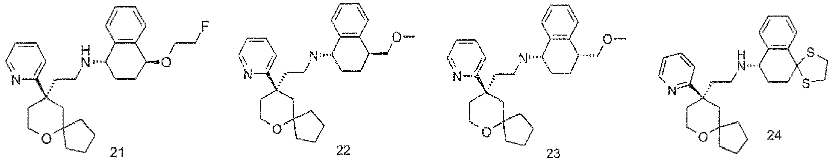 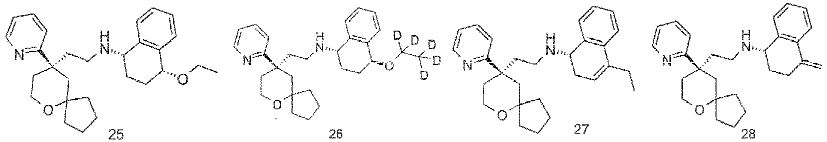 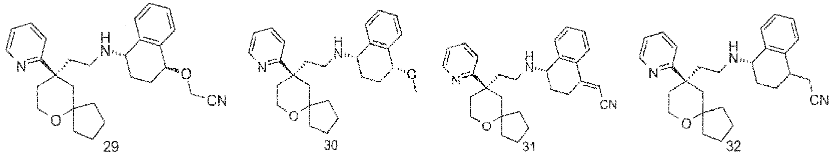 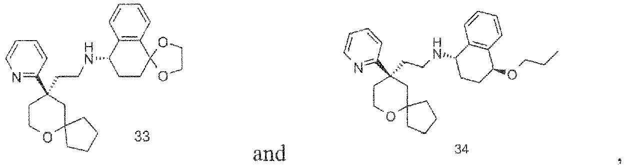 and more preferably

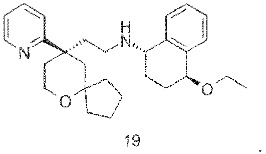

The KOR agonist is selected from a compound of formula (II): 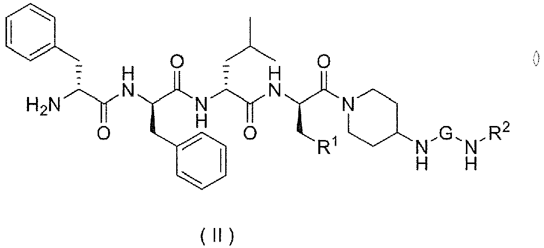 or a tautomer, mesomer, racemate, enantiomer, diastereomer thereof, or mixture thereof, or a pharmaceutically acceptable salt thereof,
wherein:
G is C=O or O=S=O;
R¹ is selected from the group consisting of hydrogen, alkyl, alkoxy, haloalkyl, halogen, amino, nitro, hydroxy, cyano, cycloalkyl, heterocyclyl, aryl, heteroaryl, -OR³, -C(O)R³, -C(O)OR³, -S(O)ₘR³ and -NR⁴R⁵, wherein the alkyl, haloalkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl, heteroaryl and NR⁶R⁷;
R² is selected from the group consisting of hydrogen, alkyl, alkoxy, haloalkyl, cycloalkyl, heterocyclyl, aryl, heteroaryl, -OR³, -C(O)R³ and -C(O)OR³, wherein the alkyl, haloalkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R³ is selected from the group consisting of hydrogen, alkyl, amino, alkoxy, hydroxyalkyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, halogen, hydroxy, amino, nitro, cyano, alkoxy, hydroxyalkyl, cycloalkyl, heterocyclyl, aryl and heteroaryl;
R⁴ and R⁵ are each independently selected from the group consisting of hydrogen, alkyl, alkoxy, hydroxyalkyl, hydroxy, amino, alkoxycarbonyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
R⁶ is hydrogen or W;
R⁷ is selected from the group consisting of hydrogen, alkyl, C(O)R⁸, C(O)OR⁸, C(O)NR⁹R¹⁰ and W, wherein the alkyl is optionally substituted by one or more substituents selected from the group consisting of alkoxy, hydroxyalkyl, cycloalkyl, heterocyclyl, carboxy and alkoxycarbonyl;
R⁸ is selected from the group consisting of hydrogen, alkyl, alkoxy, amino, cycloalkyl and heterocyclyl, wherein the heterocyclyl is optionally substituted by one or more substituents selected from the group consisting of alkyl, alkoxy, hydroxyalkyl, cycloalkyl, heterocyclyl, carboxy and alkoxycarbonyl;
R⁹ and R¹⁰ are each independently selected from the group consisting of hydrogen, alkyl and haloalkyl, wherein the alkyl is optionally substituted by one or more substituents selected from the group consisting of carboxy, alkoxy, amino, cyano, nitro, hydroxy, hydroxyalkyl, cycloalkyl, heterocyclyl, aryl and heteroaryl;
or R⁹ and R¹⁰ together with the nitrogen atom to which they are attached form a heterocyclyl, wherein the heterocyclyl comprises one or two identical or different heteroatoms selected from the group consisting of N, O and S, and the heterocyclyl is optionally substituted by one or more substituents selected from the group consisting of alkyl, alkoxy, halogen, amino, cyano, nitro, hydroxy, hydroxyalkyl, cycloalkyl, heterocyclyl, carboxy, alkoxycarbonyl, aryl and heteroaryl;
W is an amino protecting group; and
m is 0, 1 or 2.

In an embodiment of the present invention, the KOR agonist is selected from a compound of formula (II-A): 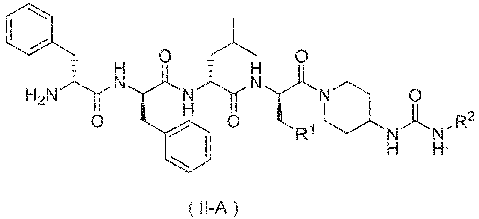 or a tautomer, mesomer, racemate, enantiomer, diastereomer thereof, or mixture thereof, or a pharmaceutically acceptable salt thereof,
wherein:
R¹ and R² are as defined in formula (II).

In another embodiment of the present invention, the KOR agonist is selected from a compound of formula (II-B): 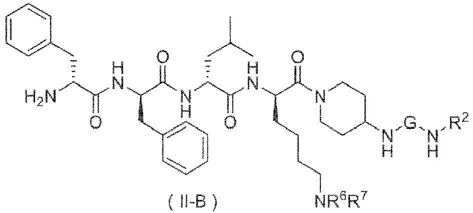 or a tautomer, mesomer, racemate, enantiomer, diastereomer thereof, or mixture thereof, or a pharmaceutically acceptable salt thereof,
wherein:
G, R⁶-R⁷ and R² are as defined in formula (II).

In an embodiment of the present invention, the KOR agonist is selected from the group consisting of: 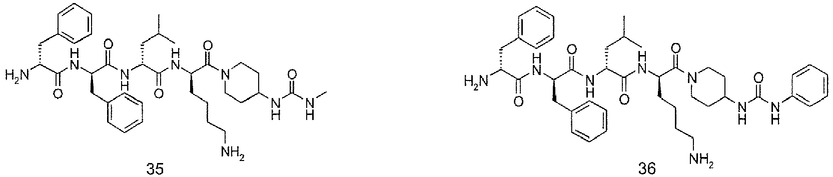 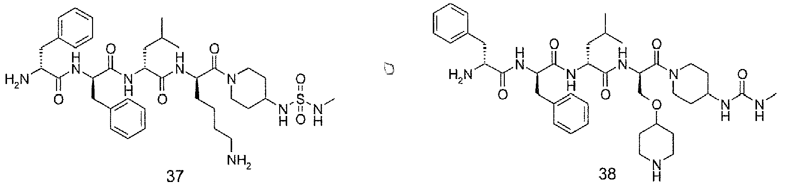 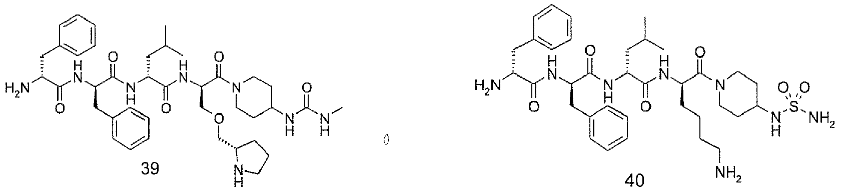 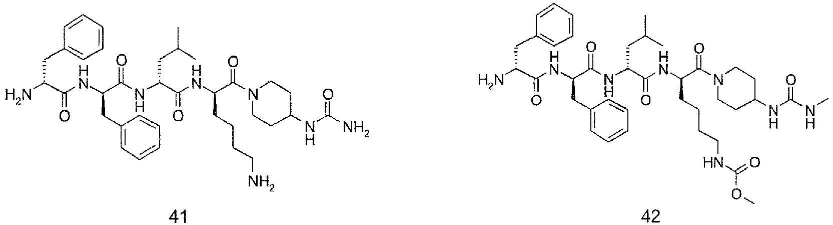 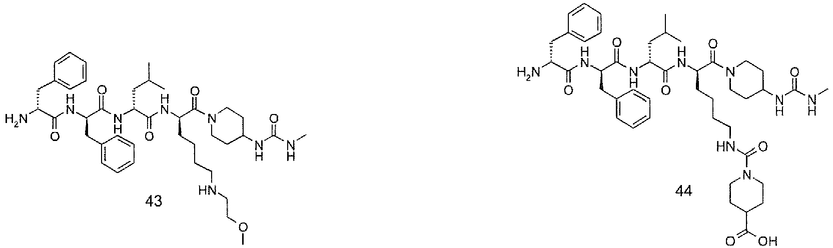 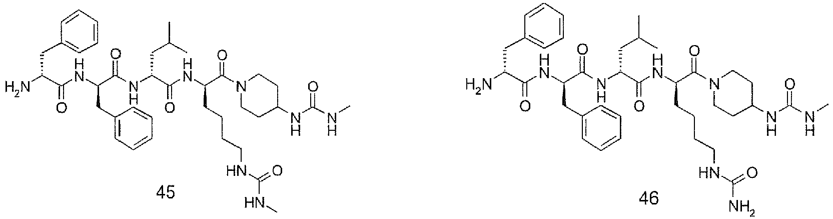 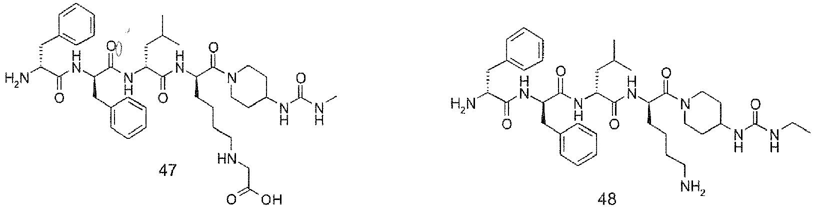 and 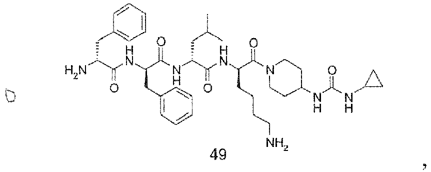 and more preferably

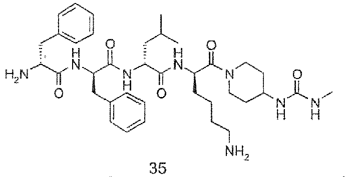

In another embodiment of the present invention, the KOR agonist is selected from the group consisting of difelikefalin, dinalbuphine, eluxadoline, nalmefene, nalfurafine, levorphanol, asimadoline, TH-030418, XE-440, CR-665, trimebutine, trimebutine-3-thiocarbamoyl-benzenesulfonate, WOL-071007, isoquinolinone 2.1, NKTR-195, 11C-FEKAP, SYK-524, nalbuphine, NRT-12, NRT-11, BU-08028, MCP-201, SA-14867, SA-14867, buprenorphine, NRT-10, GR-44821, MGM-9, KT-95, RDC-5768, GR-89696, U-50488, spiradoline, Xen-6205, LPK-26, cocaine, EN-3231, ADL-10-0101, DuP-747, fedotozine, ADL-10-0116, apadoline, ICI-204448, CJ-15161, FE-200041, ICI-199441, TAN-684, enadoline, ADL-1-0386, R-84760, E-2078, GR-103545, GR-91272, GR-86014, SEP-130551, SB-215519, SB-215520, niravoline, GR-102908, RP-61127, GR-107537, GR-129083, GR-38414, BRL-52656, GR-94839, GR-45809, EMD-60400, BRL-53001, BRL-53114, ZT-52537 and N-CBM-TAMO, preferably difelikefalin, levorphanol, asimadoline and nalfurafine, and more preferably nalfurafine.

In the above embodiments, the combination of the MOR agonist and the KOR agonist has a synergistic effect on alleviating and/or treating pain. Preferably, the combination of compound 19 or a pharmaceutically acceptable salt thereof and compound 35 or a pharmaceutically acceptable salt thereof has a synergistic effect on alleviating and/or treating pain, and the combination of compound 19 or a pharmaceutically acceptable salt thereof and nalfurafine has a synergistic effect on alleviating and/or treating pain.

The present invention provides a method for alleviating and/or treating pain, comprising administrating to a patient the above MOR agonist and KOR agonist.

According to the use of the present invention, the pain is selected from the group consisting of acute pain and chronic pain, and the chronic pain is selected from the group consisting of headache, maxillofacial pain, cervical occipital pain, neck and shoulder pain, upper limb pain, chest pain, abdominal pain, lumbocrural pain, genital tract pain, urinary tract pain and dysmenorrhea.

According to the use of the present invention, the pain is selected from the group consisting of traumatic pain, inflammatory pain, ischemic pain, pain caused by metabolic diseases, neuropathic pain, pain caused by tissue and organ malformation, labor pain and pain caused by malignant proliferative diseases.

According to the use of the present invention, the traumatic pain is selected from the group consisting of pain caused by surgery (for example postoperative pain caused by appendectomy, open colorectal surgery, hernia repair, prostatectomy, colonectomy, gastrectomy, splenectomy, colectomy, colostomy, pelvic abdominoscopy, tubal ligation, hysterectomy, vasectomy or cholecystectomy), pain after medical treatment (for example pain after colonoscopy, cystoscopy, hysteroscopy, or cervical or endometrial biopsy), fracture pain, burn pain, abdominal traumatic pain, spinal traumatic pain, chest traumatic pain and post-traumatic headache.

According to the use of the present invention, the inflammatory pain is selected from the group consisting of inflammatory headache, tissue inflammatory pain (for example rheumatoid arthritis, rheumatic arthritis, osteoarthritis), organ and gland inflammatory pain (for example gastroesophageal reflux disease, pancreatitis, acute pyelonephritis, ulcerative colitis, cholecystitis, cirrhosis, hepatic cyst, hepatitis, duodenal ulcer or gastric ulcer, esophagitis, gastritis, gastroenteritis, colitis, diverticulitis, intestinal obstruction, ovarian cyst, pelvic inflammatory disease, ulcer perforation, peritonitis, prostatitis, interstitial cystitis) and vascular inflammatory pain.

According to the use of the present invention, the ischemic pain is selected from the group consisting of ischemic headache, limb ischemic pain, tissue ischemic pain, and organ and gland ischemic pain.

According to the use of the present invention, the pain caused by metabolic diseases is selected from the group consisting of pain caused by gout and pain caused by diabetes.

According to the use of the present invention, the neuropathic pain is selected from the group consisting of phantom limb pain, stump pain, burning neuralgia, postherpetic neuralgia, sympathetic-related pain, pain caused by burning foot syndrome, folic acid deficiency peripheral neuralgia, vitamin B12 deficiency peripheral neuralgia, vitamin B1 deficiency multiple neuralgia and leprosy neuralgia.

According to the use of the present invention, the pain caused by malignant proliferative diseases is pain caused by tumors, including but not limited to pain caused by leukemia, lymphoma, myeloma, breast cancer, lung cancer, esophageal cancer, stomach cancer, colorectal cancer, liver cancer, pancreatic cancer, head and neck cancer, kidney cancer, bladder cancer, prostate cancer, ovarian cancer, endometrial cancer, cervical cancer, osteosarcoma, soft tissue sarcoma, melanoma, brain tumor.

According to the use of the present invention, the pain is a pain with a score of 4 to 10 by the VAS pain scale. Preferably, the pain with a score of 4 to 10 by the VAS pain scale is selected from the group consisting of traumatic pain, labor pain, pain caused by tumors and inflammatory pain.

According to the use of the present invention, the pain with a score of 4 to 10 by the VAS pain scale is not applicable and/or not sensitive to non-opioid analgesics or weak opioid analgesics.

The present invention provides a combination of the above MOR agonist and the above KOR agonist for use as a medicament for alleviating and/or treating pain.

According to the use of the present invention, the weight ratio of the MOR agonist to the KOR agonist is 0.001-100, preferably 1/1000, 1/750, 1/500, 1/250, 1/100, 1/90, 1/80, 1/75, 1/70, 1/60, 1/50, 1/40, 1/30, 7/30, 1/20, 7/20, 3/20, 9/20, 1/25, 2/25, 3/25, 4/25, 6/25, 7/25, 8/25, 9/25, 18/25, 1/15, 2/15, 4/15, 1/18, 5/18, 7/18, 1/14, 3/14, 5/14, 9/14, 1/12, 5/12, 7/12, 1/10, 3/10, 7/10, 9/10, 1/9, 2/9, 4/9, 1/8, 3/8, 5/8, 1/7, 2/7, 3/7, 4/7, 5/7, 6/7, 1/6, 5/6, 1/5, 2/5, 3/5, 4/5, 1/4, 3/4, 1/3, 2/3, 1/2, 1/1, 2/1, 3/1, 5/1, 10/1, 20/1, 25/1, 30/1, 50/1, and further preferably 1/1, 3/5, 1/2, 7/15, 2/5, 1/3, 3/10, 4/15, 1/4, 2/9, 1/5, 1/6, 4/25, 3/20, 2/15, 1/8, 1/9, 1/10, 1/12, 2/25, 1/15, 1/20, 1/25, 1/30, 1/40, 1/50, 1/60, 1/70, 1/75, 1/80, 1/90, 1/100.

According to the use of the present invention, the administration dose of the MOR agonist is 0.001-50 mg, and preferably 0.001 mg, 0.002 mg, 0.003 mg, 0.004 mg, 0.005 mg, 0.01 mg, 0.02 mg, 0.03 mg, 0.05 mg, 0.1 mg, 0.15 mg, 0.2 mg, 0.25 mg, 0.3 mg, 0.4 mg, 0.5 mg, 0.6 mg, 0.75 mg, 0.8 mg, 0.9 mg, 1 mg, 1.25 mg, 1.5 mg, 1.75 mg, 2 mg, 2.25 mg, 2.5 mg, 2.75 mg, 3 mg, 3.25 mg, 3.5 mg, 3.75 mg, 4 mg, 4.5 mg, 5 mg, 6 mg, 7 mg, 7.5 mg, 8 mg, 9 mg, 10 mg, 12.5 mg, 15 mg, 17.5 mg, 20 mg, 25 mg, 30 mg, 40 mg, 50 mg; the administration dose of the KOR agonist is 0.001-250 mg, and preferably 0.005 mg, 0.01 mg, 0.05 mg, 0.1 mg, 0.15 mg, 0.2 mg, 0.25 mg, 0.3 mg, 0.4 mg, 0.5 mg, 0.6 mg, 0.75 mg, 0.8 mg, 0.9 mg, 1 mg, 1.25 mg, 1.5 mg, 1.75 mg, 2 mg, 2.25 mg, 2.5 mg, 2.75 mg, 3 mg, 3.25 mg, 3.5 mg, 3.75 mg, 4 mg, 4.5 mg, 5 mg, 6 mg, 7 mg, 7.5 mg, 8 mg, 9 mg, 10 mg, 12.5 mg, 15 mg, 17.5 mg, 18 mg, 20 mg, 25 mg, 30 mg, 40 mg, 50 mg, 75 mg, 100 mg, 125 mg, 150 mg, 175 mg, 200 mg, 250 mg.

According to the use of the present invention, further preferably, the MOR agonist is compound 19 or a pharmaceutically acceptable salt thereof, the administration dose is 0.001-20 mg, and preferably 0.001 mg, 0.002 mg, 0.003 mg, 0.004 mg, 0.005 mg, 0.01 mg, 0.02 mg, 0.03 mg, 0.1 mg, 0.15 mg, 0.2 mg, 0.25 mg, 0.3 mg, 0.4 mg, 0.5 mg, 0.6 mg, 0.75 mg, 0.8 mg, 0.9 mg, 1 mg, 1.25 mg, 1.5 mg, 1.75 mg, 2 mg, 2.25 mg, 2.5 mg, 2.75 mg, 3 mg, 3.25 mg, 3.5 mg, 3.75 mg, 4 mg, 4.5 mg, 5 mg, 6 mg, 7 mg, 7.5 mg, 8 mg, 9 mg, 10 mg, 12.5 mg, 15 mg, 17.5 mg, 20 mg.

According to the use of the present invention, further preferably, the KOR agonist is selected from the group consisting of compound 35 or a pharmaceutically acceptable salt thereof and nalfurafine, the administration dose is 0.001-100 mg, and preferably 0.005 mg, 0.01 mg, 0.05 mg, 0.1 mg, 0.15 mg, 0.2 mg, 0.25 mg, 0.3 mg, 0.4 mg, 0.5 mg, 0.6 mg, 0.75 mg, 0.8 mg, 0.9 mg, 1 mg, 1.25 mg, 1.5 mg, 1.75 mg, 2 mg, 2.25 mg, 2.5 mg, 2.75 mg, 3 mg, 3.25 mg, 3.5 mg, 3.75 mg, 4 mg, 4.5 mg, 5 mg, 6 mg, 7 mg, 7.5 mg, 8 mg, 9 mg, 10 mg, 12.5 mg, 15 mg, 17.5 mg, 18 mg, 20 mg, 25 mg, 30 mg, 40 mg, 50 mg, 75 mg, 100 mg.

According to the use of the present invention, the administration dose of the MOR agonist is 0.01-500 µg/kg, and preferably 0.01 µg/kg, 0.05 µg/kg, 0.1 µg/kg, 0.2 µg/kg, 0.25 µg/kg, 0.3 µg/kg, 0.4 µg/kg, 0.5 µg/kg, 0.6 µg/kg, 0.7 µg/kg, 0.8 µg/kg, 0.9 µg/kg, 1 µg/kg, 2 µg/kg, 2.5 µg/kg, 3 µg/kg, 4 µg/kg, 5 µg/kg, 8 µg/kg, 10 µg/kg, 15 µg/kg, 20 µg/kg, 24 µg/kg, 25 µg/kg, 30 µg/kg, 40 µg/kg, 50 µg/kg, 60 µg/kg, 70 µg/kg, "5 µg/kg, 80 µg/kg, 90 µg/kg, 100 µg/kg, 200 µg/kg, 250 µg/kg, 300 µg/kg, 400 µg/kg, 500 µg/kg; the administration dose of the KOR agonist is 0.01-500 µg/kg, and preferably 0.01 µg/kg, 0.05 µg/kg, 0.1 µg/kg, 0.2 µg/kg, 0.25 µg/kg, 0.3 µg/kg, 0.4 µg/kg, 0.5 µg/kg, 0.6 µg/kg, 0.7 µg/kg, 0.8 µg/kg, 0.9 µg/kg, 1 µg/kg, 2 µg/kg, 2.5 µg/kg, 3 µg/kg, 4 µg/kg, 5 µg/kg, 8 µg/kg, 10 µg/kg, 15 µg/kg, 20 µg/kg, 24 µg/kg, 25 µg/kg, 30 µg/kg, 40 µg/kg, 50 µg/kg, 60 µg/kg, 70 µg/kg, 75 µg/kg, 80 µg/kg, 90 µg/kg, 100 µg/kg, 200 µg/kg, 250 µg/kg, 300 µg/kg, 400 µg/kg, 500 µg/kg.

According to the use of the present invention, the MOR agonist is compound 19 or a pharmaceutically acceptable salt thereof, the administration dose is 0.01-150 µg/kg, and preferably 0.01 µg/kg, 0.05 µg/kg, 0.1 µg/kg, 0.2 µg/kg, 0.25 µg/kg, 0.3 µg/kg, 0.4 µg/kg, 0.5 µg/kg, 0.6 µg/kg, 0.7 µg/kg, 0.8 µg/kg, 0.9 µg/kg, 1 µg/kg, 1.5 µg/kg, 2 µg/kg, 2.5 µg/kg, 3 µg/kg, 4 µg/kg, 5 µg/kg, 8 µg/kg, 10 µg/kg, 15 µg/kg, 20 µg/kg, 24 µg/kg, 25 µg/kg.

According to the use of the present invention, the KOR agonist is selected from the group consisting of compound 35 or a pharmaceutically acceptable salt thereof and nalfurafine, the administration dose is 0.01-150 µg/kg, and preferably 0.01 µg/kg, 0.05 µg/kg, 0.1 µg/kg, 0.2 µg/kg, 0.25 µg/kg, 0.3 µg/kg, 0.4 µg/kg, 0.5 µg/kg, 0.6 µg/kg, 0.7 µg/kg, 0.8 µg/kg, 0.9 µg/kg, 1 µg/kg, 2 µg/kg, 2.5 µg/kg, 3 µg/kg, 4 µg/kg, 5 µg/kg, 8 µg/kg, 10 µg/kg, 15 µg/kg, 20 µg/kg, 24 µg/kg, 25 µg/kg, 50 µg/kg, 75 µg/kg, 100 µg/kg.

The administration mode of the combination of the present invention is selected from the group consisting of: simultaneous administration, co-administration after separate formulation, and sequential administration after separate formulation.

The present invention further relates to a use of a combination of a MOR agonist and a KOR agonist in the preparation of a medicament for alleviating and/or treating pain, wherein the initial administration dose of the MOR agonist is 1-50 times the maintenance dose, and the initial administration dose of the KOR agonist is 0.05-50 times the maintenance dose.

The present invention further relates to a use of a combination of a MOR agonist and a KOR agonist in the preparation of a medicament for alleviating and/or treating pain, wherein the administration frequency of the MOR agonist is once a day, twice a day, three times a day, once a week, once every two weeks, once every three weeks, once a month, and the administration frequency of the KOR agonist is once a day, twice a day, three times a day, once a week, once every two weeks, once every three weeks, once a month.

In the above embodiments, the combination also optionally comprises a third component selected from the group consisting of an opioid, glucocorticoid, non-steroidal anti-inflammatory drug, local anesthetic, anti-depressant, calcium channel antagonist, anti-convulsant, adrenal beta blocker, anesthetic, and anesthesia inducer.

The present invention also relates to a pharmaceutical composition of a MOR agonist and a KOR agonist comprising optional one or more pharmaceutically acceptable carriers, excipients and/or diluents. The pharmaceutical composition can be formulated into any one of the pharmaceutically acceptable dosage forms. For example, a pharmaceutical formulation comprising a MOR agonist and a KOR agonist as the active ingredients can be formulated into a tablet, capsule, pill, granule, solution, suspension, syrup, injection (including injection solution, sterile powder for injection and concentrated solution for injection), suppository, inhalant or spray.

The pharmaceutical composition of the MOR agonist and the KOR agonist of the present invention can be administrated alone, or in combination with one or more therapeutic agents.

The components to be combined (for example, the MOR agonist and the KOR agonist, the MOR agonist and the KOR agonist and the optional third component) can be administrated simultaneously or sequentially separately. Moreover, the components to be combined can also be co-administrated in the same formulation or in separately different formulations.

In the present invention, the term "combined administration" or "co-administration" is an administration mode, including various situations in which the two or more drugs are administrated sequentially or simultaneously. The term "simultaneously" herein means that the MOR agonist and the KOR agonist, or the MOR agonist and the KOR agonist and the optional third component are administered during the same administration cycle, for example, the two or more drugs are administrated within one day, three days, one week, two weeks or one month. The term "sequential or successive" administration includes situations in which the MOR agonist and the KOR agonist, or the MOR agonist and the KOR agonist and the optional third component are administrated respectively, in different administration cycles. These administration modes all belong to the combined administration of the present invention.

The term "effective amount" of the present invention encompasses an amount sufficient to ameliorate or prevent a symptom or sign of a medical condition. The term "effective amount" also refers to an amount sufficient to allow or facilitate diagnosis. An effective amount for a particular patient or veterinary subject may vary depending on factors such as the condition to be treated, the general health of the patient, the route and dose of administration, and the severity of side effects. An effective amount can be the maximal dose or administration regimen that avoids significant side effects or toxic effects.

### DEFINITIONS

In the specification and claims of the present application, unless otherwise indicated, the scientific and technical terms used herein have the meanings generally understood by a person skilled in the art. However, in order to understand the present invention better, definitions and explanations of some related terms are provided. In addition, when the definitions and explanations of the terms provided in the present application are inconsistent with the meanings generally understood by a person skilled in the art, the definitions and explanations of the terms provided in the present application shall prevail.

The term "hydroxyalkyl" refers to an alkyl group substituted by hydroxy(s), wherein the alkyl is as defined above.

The term "haloalkyl" refers to an alkyl group substituted by one or more halogens, wherein the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "carboxy" refers to a -C(O)OH group.

The term "alkoxycarbonyl" refers to a -C(O)O(alkyl) or -C(O)O(cycloalkyl) group, wherein the alkyl and cycloalkyl are as defined above.

All of "X is selected from the group consisting of A, B, or C", "X is selected from the group consisting of A, B and C", "X is A, B or C", "X is A, B and C" and the like, are of the same meaning, i.e., X can be any one or more of A, B, and C.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without paying excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present invention, which is safe and effective in mammals and has the desired biological activity.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio. heterocyclylthio, oxo, carboxy and alkoxycarbonyl.

The term "cycloalkyl" refers to a saturated or partially unsaturated monocyclic or polycyclic hydrocarbon substituent group having 3 to 20 carbon atoms, preferably 3 to 12 carbon atoms, more preferably 3 to 6 carbon atoms, and most preferably 5 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably 6 to 14 membered spiro cycloalkyl, and more preferably 7 to 10 membered spiro cycloalkyl. According to the number of the spiro atoms shared between the rings, the spiro cycloalkyl can be divided into mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

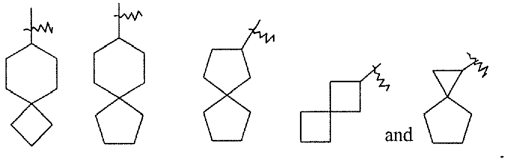

The term "fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated π-electron system. The fused cycloalkyl is preferably 6 to 14 membered fused cycloalkyl, and more preferably 7 to 10 membered fused cycloalkyl. According to the number of membered rings, the fused cycloalkyl can be divided into bicyclic, tricyclic, tetracyclic or polycyclic fused cycloalkyl, and the fused cycloalkyl is preferably bicyclic or tricyclic fused cycloalkyl, and more preferably 5-membered/5-membered, or 5-membered/6-membered bicyclic fused cycloalkyl. Non-limiting examples of fused cycloalkyl include:

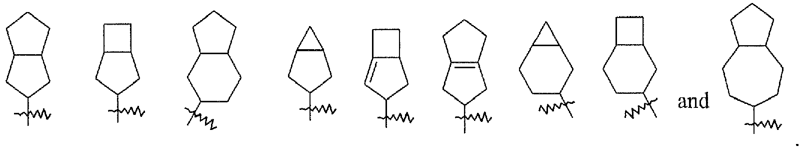

The term "bridged cycloalkyl" refers to a 5 to 20 membered all-carbon polycyclic group, wherein every two rings in the system share two disconnected carbon atoms, wherein the rings can have one or more double bonds, but none of the rings has a completely conjugated π-electron system. The bridged cycloalkyl is preferably 6 to 14 membered bridged cycloalkyl, and more preferably 7 to 10 membered bridged cycloalkyl. According to the number of membered rings, the bridged cycloalkyl can be divided into bicyclic, tricyclic, tetracyclic or polycyclic bridged cycloalkyl, and the bridged cycloalkyl is preferably bicyclic, tricyclic or tetracyclic bridged cycloalkyl, and more preferably bicyclic or tricyclic bridged cycloalkyl. Non-limiting examples of bridged cycloalkyls include:

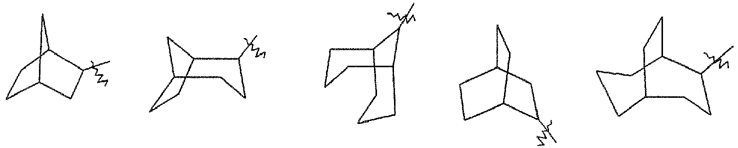

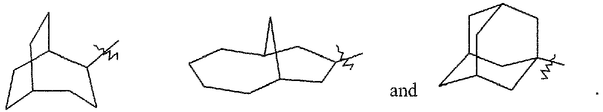

The ring of cycloalkyl can be fused to the ring of aryl, heteroaryl or heterocyclyl, wherein the ring bound to the parent structure is cycloalkyl. Non-limiting examples include indanyl, tetrahydronaphthyl, benzocycloheptyl and the like, and preferably benzocyclopentyl, tetrahydronaphthyl. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, the heterocyclyl has 3 to 8 ring atoms wherein 1 to 3 atoms are heteroatoms; and most preferably 5 to 6 ring atoms wherein 1 to 2 or 1 to 3 atoms are heteroatoms. Non-limiting examples of monocyclic heterocyclyl include pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and the like, and preferably tetrahydropyranyl, piperidinyl, pyrrolidinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and S(O)ₘ (wherein m is an integer of 0 to 2), with the remaining ring atoms being carbon atoms, where the rings can contain one or more double bonds, but none of the rings has a completely conjugated π-electron system. The spiro heterocyclyl is preferably 6 to 14 membered spiro heterocyclyl, and more preferably 7 to 10 membered spiro heterocyclyl. According to the number of the spiro atoms shared between the rings, the spiro heterocyclyl can be divided into mono-spiro heterocyclyl, di-spiro heterocyclyl, or poly-spiro heterocyclyl, and the spiro heterocyclyl is preferably mono-spiro heterocyclyl or di-spiro heterocyclyl, and more preferably 4-membered/4-membered, 4-membered/5-membered, 4-membered/6-membered, 5-membered/5-membered, or 5-membered/6-membered mono-spiro heterocyclyl. Non-limiting examples of spiro heterocyclyls include:

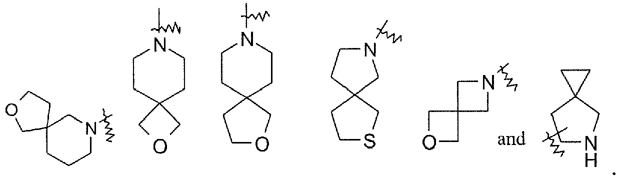

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably 6 to 14 membered fused heterocyclyl, and more preferably 7 to 10 membered fused heterocyclyl. According to the number of membered rings, the fused heterocyclyl can be divided into bicyclic, tricyclic, tetracyclic or polycyclic fused heterocyclyl, and the fused heterocyclyl is preferably bicyclic or tricyclic fused heterocyclyl, and more preferably 5-membered/5-membered or 5-membered/6-membered bicyclic fused heterocyclyl. Non-limiting examples of fused heterocyclyl include:

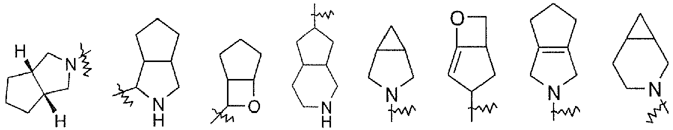

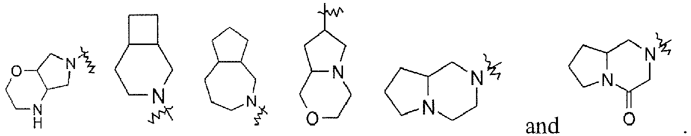

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The bridged heterocyclyl is preferably 6 to 14 membered bridged heterocyclyl, and more preferably 7 to 10 membered bridged heterocyclyl. According to the number of membered rings, the bridged heterocyclyl can be divided into bicyclic, tricyclic, tetracyclic or polycyclic bridged heterocyclyl, and the bridged heterocyclyl is preferably bicyclic, tricyclic or tetracyclic bridged heterocyclyl, and more preferably bicyclic or tricyclic bridged heterocyclyl. Non-limiting examples of bridged heterocyclyls include:

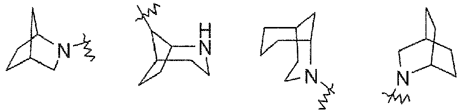

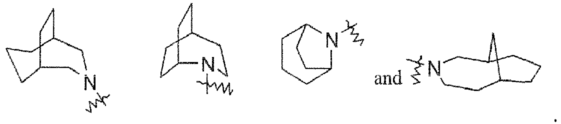

The ring of heterocyclyl can be fused to the ring of aryl, heteroaryl or cycloalkyl, wherein the ring bound to the parent structure is heterocyclyl. Non-Lmiting examples thereof include: 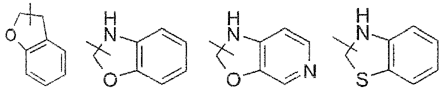 and the like.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio, oxo, carboxy and alkoxycarbonyl.

The term "aryl" refers to a 6 to 14 membered all-carbon monocyclic ring or polycyclic fused ring (i.e. each ring in the system shares an adjacent pair of carbon atoms with another ring in the system) having a conjugated π-electron system, preferably 6 to 10 membered aryl, and more preferably 5 to 6 membered aryl, for example, phenyl and naphthyl. The ring of aryl can be fused to the ring of heteroaryl, heterocyclyl or cycloalkyl, wherein the ring bound to the parent structure is aryl ring. Non-limiting examples thereof include:

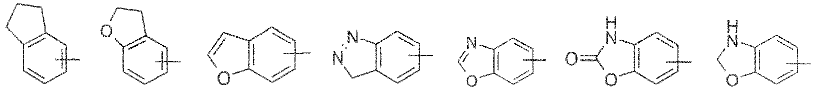

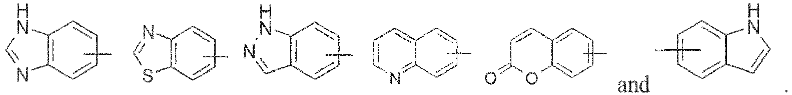

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol hydroxy, nitro, cyano, cycloalkyl, heterocyclyl, aryl, heteroaryl, cycloalkoxy, heteroalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of O, S and N. The heteroaryl is preferably 5 to 10 membered heteroaryl having 1 to 3 heteroatoms, more preferably 5 or 6 membered heteroaryl having 1 to 2 heteroatoms, preferably for example, imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, pyrazolyl, pyrimidinyl, thiazolyl, and more preferably pyrazolyl. The ring of heteroaryl can be fused to the ring of aryl, heterocyclyl or cycloalkyl, wherein the ring bound to the parent structure is heteroaryl ring. Non-limiting examples thereof include:

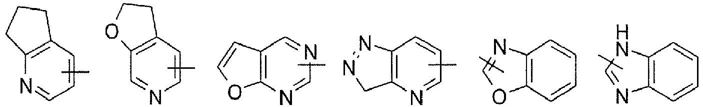

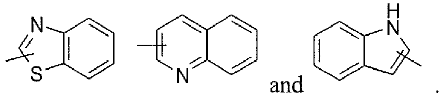

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio, carboxy and alkoxycarbonyl.

The term "amino protecting group" refers to a group suitable for protecting (preventing) an amino group from a chemical reaction, and it is easily removed after completion of a chemical reaction at other parts of the molecule. Typical representatives of these groups include unsubstituted or substituted acyl, unsubstituted or substituted allyl, aryl, arylalkoxymethyl, arylalkyl, or heterocyclyl formed together with a nitrogen atom and salt. Non-limiting examples of amino protecting group include tert-butoxycarbonyl (Boc), benzyloxycarbonyl, isobutoxycarbonyl, fluorenylmethoxycarbonyl (Fmoc), benzoyl, substituted benzoyl, butyryl, acetyl, trifluoroacetyl, phthaloyl (Pht), succinimidyl, maleimido, benzyl, allyloxycarbonyl, p-methoxybenzyl and the like. These groups can be optionally substituted by one or more substituents selected from the group consisting of halogen, alkyl, alkoxy, hydroxy, nitro, acylamino, benzyl substituted by acyl and the like, o-methylbenzyl, trityl and diphenylmethyl. The amino protecting group is preferably tert-butoxycarbonyl and fluorenylmethoxycarbonyl (Fmoc).

The term "synergistic effect" includes additive effect, potentiating effect, sensitizing effect. The "synergistic effect" of the present invention includes but is not limited to reducing the tolerance when the KOR agonist or the MOR agonist is used alone, reducing the dose when the KOR agonist or the MOR agonist is used alone, reducing the side effects when the KOR agonist or the MOR agonist is used alone, improving the effect of alleviating and/or treating pain when the same dose of the KOR agonist and/or the same dose of the MOR agonist is used alone.

The term "acute pain" refers to a pain caused by harmful irritation resulting from injury and/or disease of skin, deep body structures or organs, or a short-term pain caused by abnormal function of muscles or organs that do not produce actual tissue injury.

The term "chronic pain" refers to a pain that continues beyond the usual course of an acute disease or a reasonable time for injury healing, a pain associated with the chronic pathological process that causes persistent pain, or a pain that recurs at a certain interval (several days, weeks, months and years). Moreover, chronic pain also includes a pain that still exists after the cure should have been reached or after the usual course of treatment.

The term "inflammatory pain" refers to a pain caused by nerve stimulation of local acute inflammation or chronic inflammation.

The term "ischemic pain" refers to a pain caused by poor blood supply to the limbs or organs.

The term "neuropathic pain" refers to a pain caused by primary or secondary injury or dysfunction or transient disturbance of the peripheral or central nervous system.

The term "pain caused by malignant proliferative diseases" refers to a pain caused by tumors and cancers resulting from the malignant proliferation of somatic cells, a pain caused by lesions resulting from the malignant proliferation of virus in human organs, glands, blood system and skin, and a pain caused by lesions resulting from the malignant proliferation of bacteria in human organs, glands, blood system and skin.

The term "tissue" refers to a population of cells that are identical or similar in morphology and identical in function, including but not limited to epithelial tissue, connective tissue, muscle tissue, neural tissue, for example cartilage tissue, bone tissue, skeletal muscle, myocardium, smooth muscle.

The term "VAS pain scale" refers to a pain evaluation criteria commonly used in clinical practice, which has an important guiding role for pain medication. The score ranges from 0 to 10, wherein: a score of 0 refers to no pain; a score below 3 refers to a slight pain that is tolerable; a score of 4 to 6 refers to a pain in patient that affects sleep and is still tolerable; a score of 7 to 10 refers to a progressively strong pain in patient that is intolerable and affects appetite and sleep.

The term "initial dose" refers to a dose administrated for the first time for eliminating clinical symptoms when a continuous administration is required.

The term "maintenance dose" refers to a dose administrated for consolidating and maintaining the efficacy after the clinical symptoms are controlled or alleviated.

The term "administration according to the need for pain" refers to an administration for the purpose of alleviating and/or treating pain according to the degree of mammalian self-perception of pain.

The meaning of the term "surgery" is not limited to the conventional definition of surgery that includes the content disclosed in the surgery classification catalogue (2011 edition) of Ministry of Health. The surgery of the present invention broadly encompasses surgical procedures that have at least one incision in the skin and mucosa, and non-conventionally defined medical procedures (for example, interventional procedures involving diagnosis and treatment).

The term "pain caused by surgery" refers to a pain response after the injury or stimulation of the surgery on the body's tissue, encompassing preoperative, intraoperative, and postoperative pain during perioperative period, including but not limited to pain after surgical procedures (for example postoperative pain caused by appendectomy, open colorectal surgery, hernia repair, prostatectomy, colonectomy, gastrectomy, splenectomy, colectomy, colostomy, pelvic abdominoscopy, tubal ligation, hysterectomy, vasectomy or cholecystectomy), pain after medical treatment (for example pain after colonoscopy, cystoscopy, hysteroscopy, or cervical or endometrial biopsy).

The term "pain caused by tumors" refers to a pain directly caused by tumors, a pain caused by the treatment of tumors, a pain indirectly caused by tumors.

### DESCRIPTION OF THE DRAWING

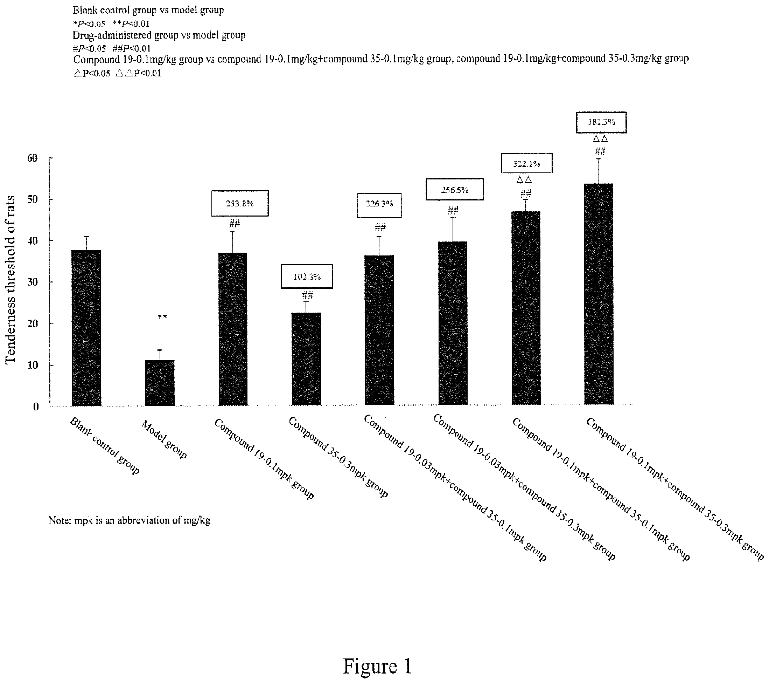

Figure 1 shows the effect of the combination of the MOR agonist and the KOR agonist (compound 19 and compound 35) of the present invention on the mechanical withdrawal threshold of rats in the incision pain test.

### DETAILED DESCRIPTION OF THE INVENTION

The exemplary experimental solutions for the use of the composition of the present invention in alleviating and/or treating pain are provided below in order to demonstrate the favorable activity and beneficial technical effects of the composition of the present invention. However, it should be understood that the following experimental solutions are merely examples of the present invention and are not intended to limit the scope of the present invention. A person skilled in the art, based on the teachings of the specification, can make suitable modifications or alterations to the technical solutions of the present invention without departing from the spirit and scope of the present invention.

### Example 1. Preparation of compound 19

Compound 19 was identified by nuclear magnetic resonance (NMR) and/or mass spectrometry (MS). NMR shifts (δ) are given in 10⁻⁶ (ppm). NMR was determined by a Bruker AVANCE-400 machine. The solvents for determination were deuterated-dimethyl sulfoxide (DMSO-*d₆*), deuterated-chloroform (CDCl₃) and deuterated-methanol (CD₃OD), and the internal standard was tetramethylsilane (TMS).

MS was determined by a FINNIGAN LCQAd (ESI) mass spectrometer (manufacturer: Thermo, type: Finnigan LCQ advantage MAX).

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm.

Yantai Huanghai 200 to 300 mesh silica gel was generally used as a carrier for column chromatography.

The known starting materials of the present invention can be prepared by the known methods in the art, or can be purchased from ABCR GmbH & Co. KG, Acros Organnics, Aldrich Chemical Company, Accela ChemBio Inc., or Dari chemical Company, etc.

Unless otherwise stated, the solution refers to an aqueous solution.

Unless otherwise stated, the reaction temperature is room temperature from 20°C to 30°C.

The reaction process in the examples was monitored by thin layer chromatography (TLC). The developing solvent system used in the reactions was dichloromethane/methanol system.

The eluent system in column chromatography and the developing solvent system in thin layer chromatography for purification of the compounds included: A: dichloromethane/methanol system, B: *n*-hexane/ethyl acetate system, and C: dichloromethane/acetone system. The ratio of the volume of the solvent was adjusted according to the polarity of the compounds, and a small quantity of alkaline reagent such as triethylamine or acidic reagent such as acetic acid can also be added for adjustment.

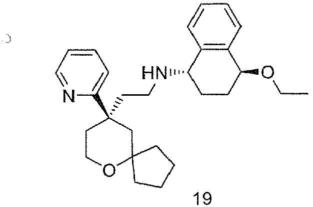

### (1S,4S)-4-Ethoxy-N-(2-((R)-9-(pyridin-2-yl)-6-oxaspiro[4.5]decan-9-yl)ethyl)-1,2,3,4-te trahydronaphthalen-1-amine

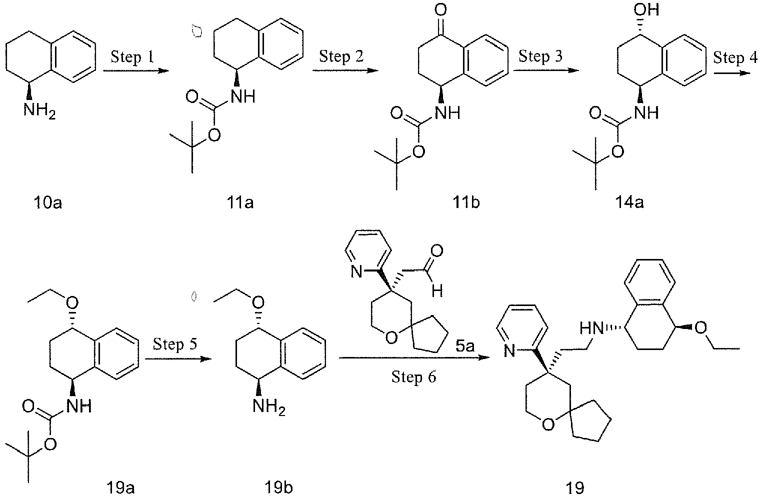

### Step 1

### (S)-Tert-butyl (1,2,3,4-tetrahydronaphthalen-1-yl)carbamate 11a

(*S*)-1,2,3,4-Tetrahydronaphthalen-1-amine **10a** (3 g, 20.41 mmol, prepared according to the known method disclosed in "Angewandte Chemie-International Edition,45(28), 4641-4644, 2006") was dissolved in 100 mL of dichloromethane, then triethylamine (5.7 mL, 40.82 mmol) and di-tert-butyl dicarbonate (4.9 g, 22. 45 mmol) were added. After stirring for 12 hours, the reaction solution was washed with water (100 mL) and saturated sodium bicarbonate solution (100 mL), successively. The organic phase was dried over anhydrous sodium sulfate and filtered. The filtrate was concentrated under reduced pressure to obtain the crude title product **11a** (5.6 g) as a light yellow oil, which was used directly in the next step without purification.
MS m/z (ESI): 248.3 [M+1]

### Step 2

### (S)-Tert-butyl (4-oxo-1,2,3,4-tetrahydronaphthalen-1-yl)carbamate 11b

The crude (*S*)-tert-butyl (1,2,3,4-tetrahydronaphthalen-1-yl)carbamate **11a** (5.6 g, 20.41 mmol) was dissolved in 90 mL of a mixed solution of acetone and water (V/V=2:1), then magnesium sulfate (5.5 g, 45.66 mmol) was added and potassium permanganate (7.22 g, 45.66 mmol) was slowly added with stirring. The reaction system was stirred for 12 hours. Then, the reaction solution was concentrated under reduced pressure, and the resulting residue was purified by silica gel column chromtography with *n*-hexane/ethyl acetate system to obtain the title product **11b** (3.1 g, yield 52%) as an off-white solid.
MS m/z (ESI): 262.3 [M+1]

### Step 3

### Tert-butyl ((1S,4S)-4-hydroxy-1,2,3,4-tetrahydronaphthalen-1-yl)carbamate 14a

(*S*)-Tert-butyl (4-oxo-1,2,3,4-tetrahydronaphthalen-1-yl)carbamate **11b** (100 mg, 0.883 mmol) was dissolved in 5 mL of toluene. The reaction solution was cooled to 0°C, added with (*R*)-2-methyl-CBS-oxazaborolidine (0.1 ml, 0.076 mmol), and stirred for 5 minutes. Then, borane methylsulfide (0.88 ml, 0.76 mmol) was added, and the reaction was stirred for 2 hours. The reaction was quenched by adding 50 ml of saturated sodium chloride solution, and extracted with ethyl acetate (30 mL×3). The organic phases were combined, washed with saturated sodium chloride solution (30 mL×3), dried over anhydrous sodium sulfate and filtered. The filtrate was concentrated under reduced pressure, and the resulting residue was purified by thin layer chromtography with dichloromethane/methanol system to obtain the title product **14a** (60 mg, yield 60%) as a white solid.
MS m/z (ESI): 208.3 [M-55]

### Step 2

### Tert-butyl ((1S,4S)-4-ethoxy-1,2,3,4-tetrahydronaphthalen-1-yl)carbamate 19a

The crude tert-butyl (1*S*)-4-hydroxy-1,2,3,4-tetrahydronaphthalen-1-yl) carbamate **14a** (850 mg, 3.23 mmol), silver oxide (76 mg, 0.33 mmol) and iodoethane (1.3 mL, 16.15 mmol) were dissolved in 30 mL of dichloromethane, and the reaction solution was stirred for 48 hours. The reaction solution was filtered, and the filtrate was concentrated under reduced pressure to obtain the crude title product **19a** (800 mg) as a yellow oil, which was used directly in the next step without purification.
MS m/z (ESI): 236.1 [M-55]

### Step 3

### (1S,4S)-4-Ethoxy-1,2,3,4-tetrahydronaphthalen-1-amine 19b

The crude compound **19a** (698 mg, 2.4 mmol) was dissolved in 4 mL of dichloromethane, then 8 mL of a solution of 4 M hydrogen chloride in 1,4-dioxane were added. After stirring for 2 hours, the reaction solution was concentrated under reduced pressure, triturated with ethyl acetate (30 mL) and filtered. The filter cake was dissolved in 20 mL of a mixed solution of dichoromethane and methanol (V:V=5:1). Saturated sodium bicarbonate solution was added to adjust the pH of the reaction solution to 7 to 8. The reaction solution was concentrated under reduced pressure, washed with a mixed solution of dichloromethane and methanol (V:V=5:1) (30 mL×2) and filtered. The filtrate was concentrated under reduced pressure to obtain the crude title product **19b** (310 mg) as a yellow liquid, which was used directly in next step without purification.
MS m/z (ESI): 191.1 [M+1]

### Step 4

### (1S,4S)-4-Ethoxy-N-(2-((R)-9-(pyridin-2-yl)-6-oxaspiro[4.5]decan-9-yl)ethyl)-1,2,3,4-te trahydronaphthalen-1-amine 19

(*R*)-2-(9-(Pyridin-2-yl)-6-oxaspiro[4.5]decan-9-yl)acetaldehyde **5a** (500 mg, 1.85 mmol, prepared according to the method disclosed in the patent application "WO2012129495") and the crude compound **19b** (310 mg, 1.85 mmol) were dissolved in 30 mL of dichloroethane, and the mixture was stirred for 40 minutes, then sodium triacetoxyborohydride (980 mg, 4.63 mmol) was added. After stirring for 2 hours, the reaction solution was washed successively with saturated sodium bicarbonate solution (30 mL×3) and saturated sodium chloride solution (30 mL×3). The organic phase was dried over anhydrous sodium sulfate and filtered. The filtrate was concentrated under reduced pressure, and the resulting residue was purified by thin layer chromatography with dichloromethane/methanol system to obtain the title product **19** (280 mg, yield 35%) as a yellow viscous solid.
MS m/z (ESI): 435.3 [M+1]
¹H NMR (400 MHz, CDCl₃) δ 9.74 (d, 1H), 9.58 (d, 1H), 8.94 (d, 1H), 8.37 (d, 1H), 7.94 (d, 1H), 7.67 (d, 1H), 7.52 (d, 1H), 7.47 (t, 1H), 4.46-4.49 (m, 1H), 4.30-4.33 (m, 1H), 3.84-3.87 (m, 1H), 3.66-3.70 (m, 2H), 3.53-3.56 (m, 2H), 2.82-2.85 (d, 2H), 2.67 (s, 2H), 2.39-2.41 (m, 4H), 2.30-2.33 (m, 4H), 1.85 (s, 2H), 1.48-1.52 (m, 6H), 1.27 (m, 3H).

### Example 2. Preparation of compound 35

The apparatus, equipment and material required for the preparation of compound 35 are shown in Example 1.

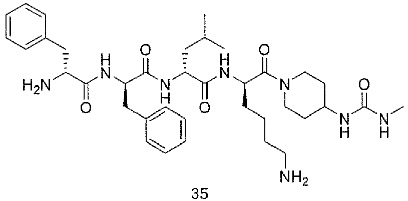

### (R)-N-((R)-6-Amino-1-(4-(3-methylureido)piperidin-1-yl)-1-oxohexan-2-yl)-2-((R)-2-(( R)-2-amino-3-phenylpropanamido)-3-phenylpropanamido)-4-methylpentanamide

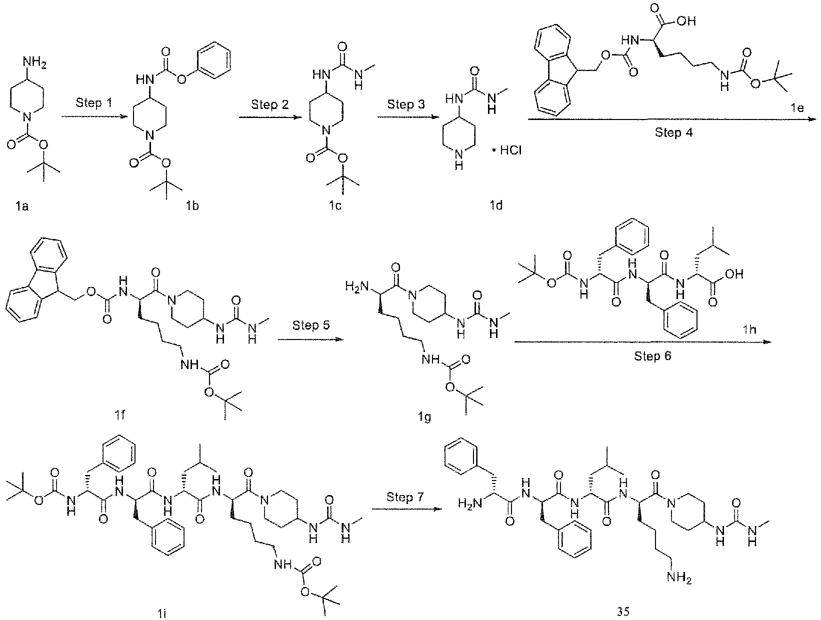

### Step 1

### Tert-butyl 4-((phenoxycarbonyl)amino)piperidine-1-carboxylate 1b

Tert-butyl 4-aminopiperidine-1-carboxylate **1a** (0.5 g, 2.5 mmol, prepared according to the method disclosed in the patent application "WO 2006115353") and pyridine (0.22 g, 2.75 mmol) were dissolved in 15 mL of tetrahydrofuran. The reaction solution was added dropwise with phenyl chloroformate (0.43 g, 2.75 mmol) at 0°C, and then warmed up to room temperature and stirred for 2 hours. The reaction solution was concentrated under reduced pressure. The residue was dissolved in ethyl acetate, washed with water, dried over anhydrous sodium sulfate and filtered. The filtrate was concentrated under reduced pressure to obtain the crude title product **1b** (0.9 g), which was used directly in next step without purification.

### Step 2

### Tert-butyl 4-(3-methylureido)piperidine-1-carboxylate 1c

The crude **1b** (0.9 g, 2.5 mmol) was dissolved in 20 mL of methanol. The reaction solution was added with 1.3 mL of a solution of 2 *M* methylamine in tetrahydrofuran, and stirred at 50°C for 12 hours. The reaction solution was cooled to room temperature, and concentrated under reduced pressure. The resulting residue was purified by thin layer chromatography with dichloromethane/methanol system to obtain the title product **1c** (0.35 g, yield 55%).
MS m/z (ESI): 256.1 [M-1]

### Step 3

### 1-Methyl-3-(piperidin-4-yl)urea hydrochloride Id

**1c** (0.35 g, 1.36 mmol) was dissolved in 5 mL of dichloromethane. The reaction solution was added with 1 mL of a solution of 4 *M* hydrogen chloride in 1,4-dioxane, and stirred for 2 hours. The reaction solution was concentrated under reduced pressure to obtain the crude title product **1d** (0.3 g), which was used directly in next step without purification.

### Step 4

### (R)-(9H-Fluoren-9-yl)methyl tert-butyl (6-(4-(3-methylureido)piperidin-1-yl)-6-oxohexane-1,5-diyl)dicarbamate 1f

The crude **1d** (0.3 g, 1.36 mmol) and (*R*)-2-((((9*H*-fluoren-9-yl)methoxy)carbonyl)amino)-6-((tert-butoxycarbonyl)amino)hex anoic acid **1e** (637 mg, 1.36 mmol, prepared according to the known method disclosed in *"*Tetrahedron, 2002, 58(27), 5427-5439") were dissolved in 5 mL of *N,N-*dimethylformamide. The reaction solution was added with 2-(7-azobenzotriazol-1-yl)-*N*,*N*,*N'*,*N'*-tetramethyluronium hexafluorophosphate (0.775 g, 2.04 mmol) and triethylamine (0.38 mL, 2.72 mmol), and stirred for 12 hours. The reaction solution was concentrated under reduced pressure, and the resulting residue was purified by thin layer chromatography with dichloromethane/methanol system to obtain the title product **1f** (390 mg, yield 47%).
MS m/z (ESI): 608.2 [M+1]

### Step 5 Tert-butyl (R)-(5-amino-6-(4-(3-methylureido)piperidin-1-yl)-6-oxohexyl)carbamate 1g

**1f** (120 mg, 0.197 mmol) was dissolved in 5 mL of dichloromethane. The reaction solution was added with 1 mL of hexahydropyridine, and stirred for 12 hours. The reaction solution was concentrated under reduced pressure, and the resulting residue was purified by thin layer chromatography with dichloromethane/methanol system to obtain **1g** (76 mg, yield 100%).
MS m/z (ESI): 386.2 [M+1]

### Step 6 Tert-butyl N-[(5R)-5-[(2R)-2-[(2R)-2-[(2R)-2-{[(tert-butoxy)carbonyl] amino} -3 -phenylpropionami do] -3-phenylpropionamido] -4-methylpentanoamide] -6- {4- [(methylcarbamoyl)amino]pi peridin-1-yl}-6-oxohexyl]carbamate 1i

**1g** (76 mg, 0.197 mmol), (6*R*,9*R*,12*R*)-6,9-dibenzyl-12-isobutyl-2,2-dimethyl-4,7,10-trioxo-3-oxa-5,8,11-triazatri decane-13-carboxylic acid **1h** (104 mg, 0.197 mmol, prepared according to the method disclosed in the patent application "US20110212882"), 2-(7-azobenzotriazol-1-yl)-*N*,*N*,*N'*,*N'*-tetramethyluronium hexafluorophosphate (112 mg, 0.296 mmol) and triethylamine (0.055 mL, 0.394 mmol) were dissolved in 5 mL of *N*,*N*-dimethylformamide. After stirring for 12 hours, the reaction solution was concentrated under reduced pressure, and the resulting residue was purified by thin layer chromatography with dichloromethane/methanol system to obtain the title product **1i** (100 mg, yield 57%).
MS m/z (ESI): 894.5 [M+1]

### Step 7

### (R)-N-((R)-6-Amino-1-(4-(3-methylureido)piperidin-1-yl)-1-oxohexan-2-yl)-2-((R)-2-(( R)-2-amino-3-phenylpropanamido)-3-phenylpropanamido)-4-methylpentanamide 35

**1i** (100 mg, 0.112 mmol) was dissolved in 5 mL of dichloromethane. The reaction solution was added with 0.5 mL of trifluoroacetic acid, and stirred for 2 hours. The reaction solution was concentrated under reduced pressure, and the resulting residue was purified by high performance liquid chromatography to obtain the title product **35** (10 mg, yield 14%).
MS m/z (ESI): 693.7 [M+1]
¹H NMR (400 MHz, DMSO-*d₆*): δ 8.42 (d, 1H), 8.19 (d, 1H), 7.39-7.29 (m, 10H), 7.22 (d, 1H), 6.10 (s, 1H), 5.12 (s, 4H), 4.73 (d, 1H), 4.41-4.37 (m, 2H), 4.09 (d, 1H), 3.74 (d, 1H), 3.27-3.24 (m, 3H), 3.02-2.96 (m, 4H), 2.70 (s, 3H), 2.16-1.90 (m, 2H), 1.85-1.55 (m, 9H), 1.51-1.25 (m, 6H), 1.00 (d, 3H), 0.96 (d, 3H).

### Example 3. Treatment effect of the combination of the MOR agonist and the KOR agonist of the present invention on incision pain in rats

### Test compounds

Compound 19 (prepared according to the method described in Example 1), compound 35 (prepared according to the method described in Example 2), anhydrous ethanol (Sinopharm Chemical Reagent Co., Ltd, CAS NO: 64-17-5, batch number: P1101615), polyoxyethylene hydrogenated castor oil (provided by Hunan ER-KANG Pharmaceutical Co., Ltd, batch number: 000220141102). The compound dose was calculated on bases.

### Test animals

Experimental male Wistar rats were purchased from Shanghai Slac Laboratory Animal Co., Ltd. The rats weighed 100-120 g when purchased, and fed at 5 rats/cage, in a condition of 12/12 hours light/dark cycle adjustment, a constant temperature of 23±1°C, a humidity of 50-60%, and free access to food and water. After purchase, the animals were subjected to an adaptive feeding for more than 3 days before the experiment was started.

### Experimental Apparatus

Electronic Von Frey: UGO BASILE, type 38450.

### Formulation of the solution of the test compound

Compound 19 was formulated with 10% anhydrous ethanol + 10% polyoxyethylene hydrogenated castor oil + 80% double distilled water. Compound 35 was formulated with normal saline.

### Test method

The rats were randomly divided into the following groups according to the body weight: blank control group (n=8), model group (n=8) and drug-administered group (n=48). The drug-administered group was divided into the following groups: compound 19-0.1mg/kg group (n=8), compound 19-0.3mg/kg group (n=8), compound 19-0.03mg/kg+compound 35-0.1mg/kg group (n=8), compound 19-0.03mg/kg+compound 35-0.3mg/kg group (n=8), compound 19-0.1mg/kg+compound 35-0.1mg/kg group (n=8), and compound 19-0.1mg/kg+compound 35-0.3mg/kg group (n=8). The model group and drug-administered group were subjected to an incision surgery. During the surgery, the rats were anesthetized with isoflurane. An incision (1 cm long) passing through the skin and fascia was made with a No. 10 surgical blade in the middle of the left hind paw. The skin was sutured with a 3-0 sterile silk surgical suture. The injured site was disinfected with antibiotic ointment and iodophor. The animals were returned to their original place to recover overnight. After 24 hours, the drug was injected through the tail vein, and the blank control group and the model group were administrated with the corresponding solvents. The group administered with compound 19 alone was intravenously injected with the corresponding dose of compound 19 and the blank solvent for formulating compound 35. The group administered with compound 35 alone was intravenously injected with the corresponding dose of compound 35 and the blank solvent for formulating compound 19. The group administered with the combination was intravenously injected with the corresponding dose of compound 35, and then intravenously injected with the corresponding dose of compound 19 after 30 minutes. The mechanical pain threshold of each group was measured by the Electronic Von Frey 30 minutes after the injection to evaluate the analgesic effect of the drug on the surgical incision pain and the intensity thereof.

### Data Representation and Statistical Processing

The experimental data were expressed as mean±standard deviation (S.D.). Statistical comparisons were performed using t test in the Excel software. The data between the model group and the blank control group were analyzed and compared to determine whether there was a significant statistical significance or not. *P<0.05 indicates that there is a significant difference between the model group and the blank control group, and ** P<0.01 indicates that there is a highly significant difference between the model group and the blank control group. #P<0.05 indicates that there is a significant difference between the drug-administered group and the model group, and ##P<0.01 indicates that there is a highly significant difference between the drug-administered group and the model group. ΔP<0.05 indicates that there is a significant difference between the drug-administered group and the compound 19-0.1mg/kg group, and ΔΔP<0.01 indicates that there is a highly significant difference between the drug-administered group and the compound 19-0.1mg/kg group.

### Experimental results

The results are shown in Figure 1.

### Experimental conclusion

The experimental results (Figure 1) showed that the tenderness threshold of the rats of the blank control group was 37.68 g, and the tenderness threshold of the model group was 11.08 g. Compared with the blank control group, the tenderness threshold of the model group was significantly decreased (P<0.01). 30 minutes after the drug administration, compared with the model group , the tenderness threshold of the compound 19-0.1mg/kg group was significantly increased (P<0.01) to 36.99 g, with an increase of 233.8%. 1 hour after the drug administration, compared with the model group, the tenderness threshold of the compound 35-0.3mg/kg group was significantly increased (P<0.01) to 22.42 g, with an increase of 102.3%.

Compared with the model group, the combination of compound 19 0.03mg/kg and compound 35 0.1mg/kg or compound 35 0.3mg/kg can significantly increase (P<0.01) the tenderness threshold of rats to 36.16 g or 39.5 g, with an increase of 226.3% or 256.5%, respectively, indicating that the combination of a low dose of compound 19 and compound 35 had a synergistic effect, and the dose of compound 19 was significantly reduced on the premise of achieving the same analgesic effect. The combination of compound 19 0.1mg/kg and compound 35 0.1mg/kg or compound 35 0.3mg/kg can significantly increase (P<0.01) the tenderness threshold of rats to 46.77 g or 53.44 g, with an increase of 322.1% or 382.3%, respectively; the analgesic effect of the combination of compound 19 and compound 35 was not only significantly superior than that of the model group, but also superior than that when compound 19 or compound 35 was administrated alone, indicating that the combination had a significant synergistic effect.

Compared with compound 19 0.1mg/kg alone, the combination of compound 19 0.03mg/kg and compound 35 0.1mg/kg or compound 35 0.3mg/kg had an equivalent (P>0.05) analgesic effect, respectively, and the combination of compound 19 0.1mg/kg and compound 35 0.1mg/kg or compound 35 0.3mg/kg had a better (P<0.05) analgesic effect. Compared with compound 35 0.3mg/kg alone, the four combinations all had a better (P<0.05) analgesic effect.

In addition, it was observed during the study that compound 19 alone produced more obvious toxic effects (such as slow movement and stiffness of limbs in rats) than compound 35 alone. The combination of compound 19 and compound 35 reduced the degree of adverse reactions because the dose of compound 19 was significantly reduced. Compared with the compound 19-0.1mg/kg group and the compound 19-0.3mg/kg group, the compound 19-0.03mg/kg+compound 35-0.1mg/kg group, the compound 19-0.03mg/kg+compound 35-0.3mg/kg group, the compound 19-0.1mg/kg+compound 35-0.1mg/kg group and the compound 19-0.1mg/kg+compound 35-0.3mg/kg group all showed the reduced adverse reactions in the test animals on the premise of achieving or surpassing the analgesic effect of compound 19 alone.

## Claims

1. Use of a combination of a µ opioid receptor (MOR) agonist and a κ opioid receptor (KOR) agonist in the preparation of a medicament for alleviating and/or treating pain, **characterized in that** the MOR agonist is selected from a compound of formula (I) 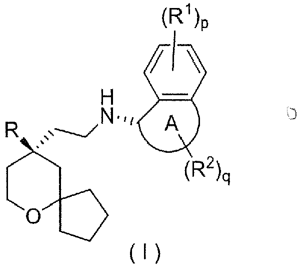 or a tautomer, mesomer, racemate, enantiomer, diastereomer thereof, or mixture thereof, or a pharmaceutically acceptable salt thereof,
wherein:
ring A is selected from the group consisting of cycloalkyl and heterocyclyl;
R is selected from the group consisting of aryl and heteroaryl, wherein the aryl and heteroaryl are each optionally substituted by one or more substituents selected from the group consisting of alkyl, haloalkyl, halogen, amino, nitro, cyano, alkoxy, haloalkoxy, hydroxyalkyl, cycloalkyl, heterocyclyl, aryl, heteroaryl, -OR³, -C(O)R³, -C(O)OR³, -S(O)ₘR³ and -NR⁴R⁵;
each R¹ is identical or different and each is independently selected from the group consisting of hydrogen, alkyl, alkoxy, haloalkyl, halogen, amino, nitro, hydroxy, cyano, cycloalkyl, heterocyclyl, aryl, heteroaryl, -OR³, -C(O)R³, -C(O)OR³, -S(O)ₘR³ and -NR⁴R⁵, wherein the alkyl, haloalkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each R² is identical or different and each is independently selected from the group consisting of hydrogen, alkyl, alkoxy, haloalkyl, halogen, amino, nitro, hydroxy, cyano, oxo, alkenyl, cycloalkyl, heterocyclyl, aryl, heteroaryl, -OR³, -C(O)R³, -C(O)OR³, -S(O)ₘR³ and -NR⁴R⁵, wherein the alkyl, alkoxy, alkenyl, haloalkyl, cycloalkyl, heterocyclyl, aryl and heteroaryl are each optionally substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl and heteroaryl;
or two R² are taken together to form a cycloalkyl or heterocyclyl, wherein the cycloalkyl or heterocyclyl is optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R³ is selected from the group consisting of hydrogen, alkyl, deuterated alkyl, amino, alkoxy, hydroxyalkyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, halogen, hydroxy, amino, nitro, cyano, alkoxy, hydroxyalkyl, cycloalkyl, heterocyclyl, aryl and heteroaryl;
R⁴ and R⁵ are each independently selected from the group consisting of hydrogen, alkyl, alkoxy, hydroxyalkyl, hydroxy, amino, alkoxycarbonyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
p and q are each independently 0, 1, 2, 3 or 4; and
m is 0, 1 or 2.

2. The use according to claim 1, **characterized in that** the MOR agonist is selected from a compound of formula (I-B): 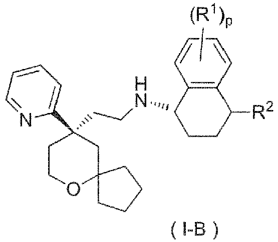 or a tautomer, mesomer, racemate, enantiomer, diastereomer thereof, or mixture thereof, or a pharmaceutically acceptable salt thereof,
wherein:
R¹, R² and p are as defined in claim 1.

3. The use according to any one of claims 1 to 2, **characterized in that** the MOR agonist is selected from the group consisting of: 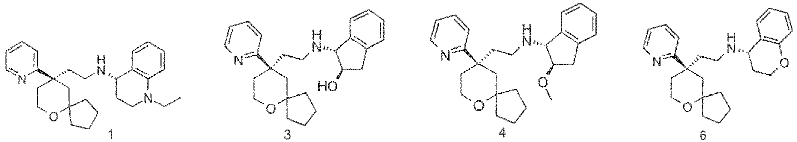 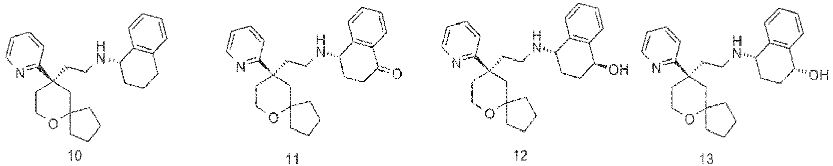 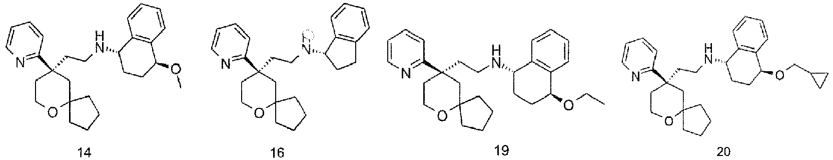 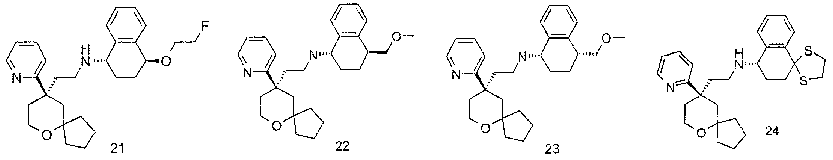 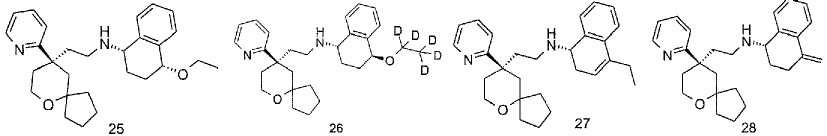 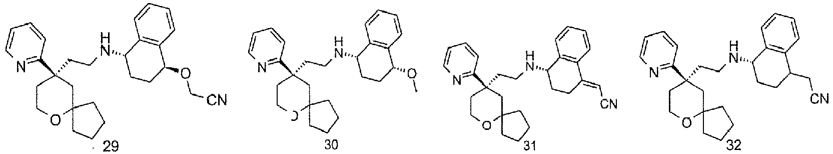 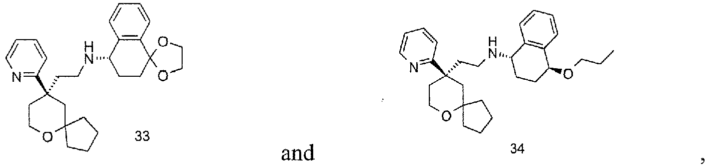 and preferably

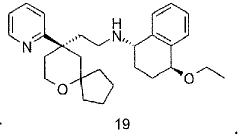

4. The use according to any one of claims 1 to 3, **characterized in that** the KOR agonist is selected from a compound of formula (II): 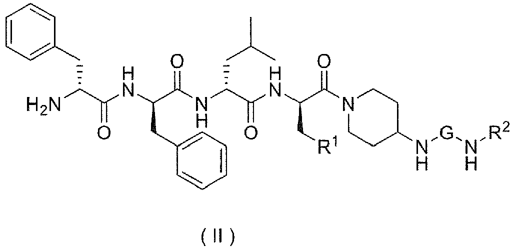 or a tautomer, mesomer, racemate, enantiomer, diastereomer thereof, or mixture thereof, or a pharmaceutically acceptable salt thereof,
wherein:
G is C=O or O=S=O;
R¹ is selected from the group consisting of hydrogen, alkyl, alkoxy, haloalkyl, halogen, amino, nitro, hydroxy, cyano, cycloalkyl, heterocyclyl, aryl, heteroaryl, -OR³, -C(O)R³, -C(O)OR³, -S(O)ₘR³ and -NR⁴R⁵, wherein the alkyl, haloalkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl, heteroaryl and NR⁶R⁷;
R² is selected from the group consisting of hydrogen, alkyl, alkoxy, haloalkyl, cycloalkyl, heterocyclyl, aryl, heteroaryl, -OR³, -C(O)R³ and -C(O)OR³, wherein the alkyl, haloalkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R³ is selected from the group consisting of hydrogen, alkyl, amino, alkoxy, hydroxyalkyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, halogen, hydroxy, amino, nitro, cyano, alkoxy, hydroxyalkyl, cycloalkyl, heterocyclyl, aryl and heteroaryl;
R⁴ and R⁵ are each independently selected from the group consisting of hydrogen, alkyl, alkoxy, hydroxyalkyl, hydroxy, amino, alkoxycarbonyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
R⁶ is hydrogen or W;
R⁷ is selected from the group consisting of hydrogen, alkyl, C(O)R⁸, C(O)OR⁸, C(O)NR⁹R¹⁰ and W, wherein the alkyl is optionally substituted by one or more substituents selected from the group consisting of alkoxy, hydroxyalkyl, cycloalkyl, heterocyclyl, carboxy and alkoxycarbonyl;
R⁸ is selected from the group consisting of hydrogen, alkyl, alkoxy, amino, cycloalkyl and heterocyclyl, wherein the heterocyclyl is optionally substituted by one or more substituents selected from the group consisting of alkyl, alkoxy, hydroxyalkyl, cycloalkyl, heterocyclyl, carboxy and alkoxycarbonyl;
R⁹ and R¹⁰ are each independently selected from the group consisting of hydrogen, alkyl and haloalkyl, wherein the alkyl is optionally substituted by one or more substituents selected from the group consisting of carboxy, alkoxy, amino, cyano, nitro, hydroxy, hydroxyalkyl, cycloalkyl, heterocyclyl, aryl and heteroaryl;
or R⁹ and R¹⁰ together with the nitrogen atom to which they are attached form a heterocyclyl, wherein the heterocyclyl comprises one or two identical or different heteroatoms selected from the group consisting of N, O and S, and the heterocyclyl is optionally substituted by one or more substituents selected from the group consisting of alkyl, alkoxy, halogen, amino, cyano, nitro, hydroxy, hydroxyalkyl, cycloalkyl, heterocyclyl, carboxy, alkoxycarbonyl, aryl and heteroaryl;
W is an amino protecting group; and
m is 0, 1 or 2.

5. The use according to claim 4, **characterized in that** the KOR agonist is selected from a compound of formula (II-A): 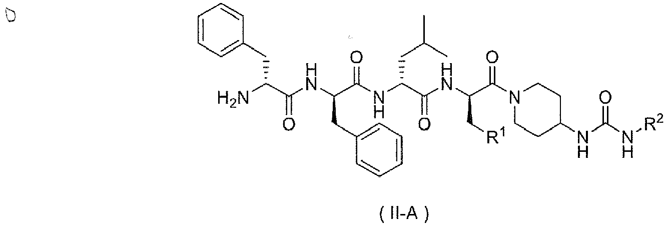 or a tautomer, mesomer, racemate, enantiomer, diastereomer thereof, or mixture thereof, or a pharmaceutically acceptable salt thereof,
wherein:
R¹ and R² are as defined in claim 4.

6. The use according to claim 4, **characterized in that** the KOR agonist is selected from a compound of formula (II-B): 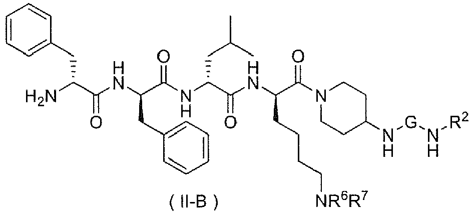 or a tautomer, mesomer, racemate, enantiomer, diastereomer thereof, or mixture thereof, or a pharmaceutically acceptable salt thereof,
wherein:
G, R⁶-R⁷ and R² are as defined in claim 4.

7. The use according to any one of claims 4 to 6, **characterized in that** the KOR agonist is selected from the group consisting of: 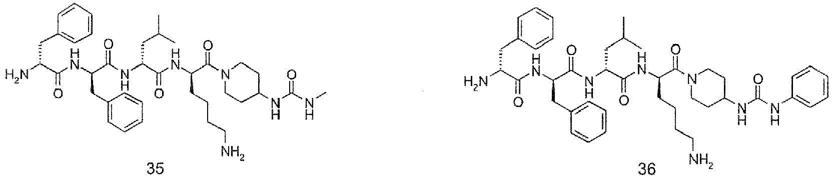 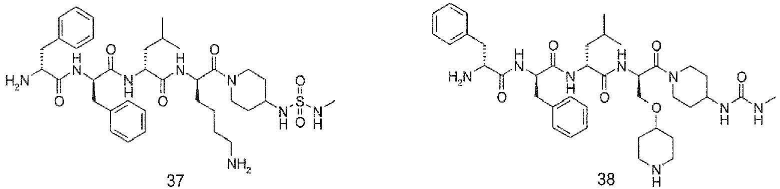 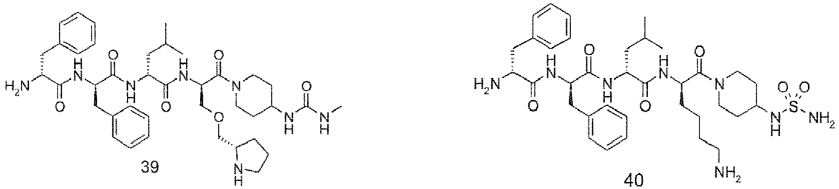 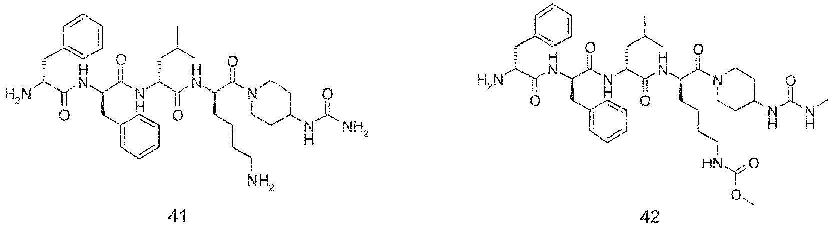 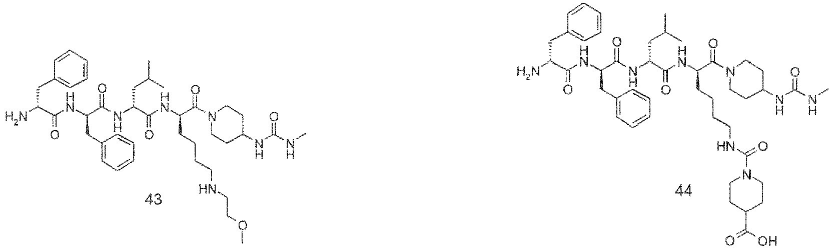 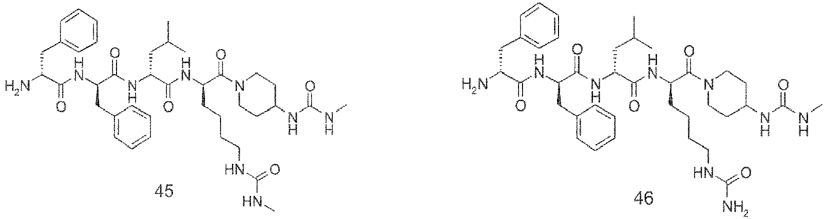 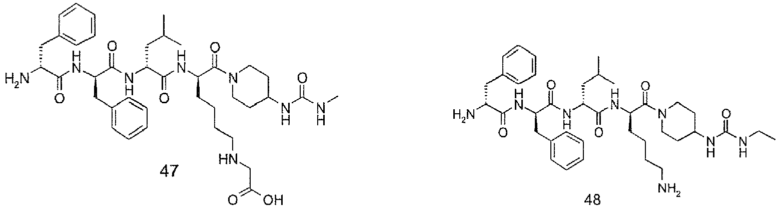 and 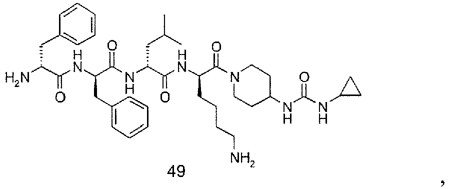 and preferably

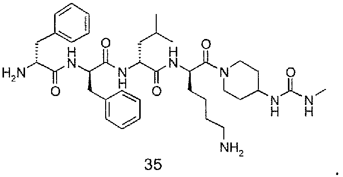

8. The use according to any one of claims 1 to 3, **characterized in that** the KOR agonist is selected from the group consisting of difelikefalin, dinalbuphine, eluxadoline, nalmefene, nalfurafine, levorphanol, asimadoline, TH-030418, XE-440, CR-665, trimebutine, trimebutine-3-thiocarbamoyl-benzenesulfonate, WOL-071007, isoquinolinone 2.1, NKTR-195, 11C-FEKAP, SYK-524, nalbuphine, NRT-12, NRT-11, BU-08028, MCP-201, SA-14867, SA-14867, buprenorphine, NRT-10, GR-44821, MGM-9, KT-95, RDC-5768, GR-89696, U-50488, spiradoline, Xen-6205, LPK-26, cocaine, EN-3231, ADL-10-0101, DuP-747, fedotozine, ADL-10-0116, apadoline, ICI-204448, CJ-15161, FE-200041, ICI-199441, TAN-684, enadoline, ADL-1-0386, R-84760, E-2078, GR-103545, GR-91272, GR-86014, SEP-130551, SB-215519, SB-215520, niravoline, GR-102908, RP-61127, GR-107537, GR-129083, GR-38414, BRL-52656, GR-94839, GR-45809, EMD-60400, BRL-53001, BRL-53114, ZT-52537 and N-CBM-TAMO, preferably difelikefalin, levorphanol, asimadoline and nalfurafine, and more preferably nalfurafine.

9. The use according to claim 1, **characterized in that** the pain is selected from the group consisting of acute pain and chronic pain, and the chronic pain is selected from the group consisting of headache, maxillofacial pain, cervical occipital pain, neck and shoulder pain, upper limb pain, chest pain, abdominal pain, lumbocrural pain, genital tract pain, urinary tract pain and dysmenorrhea.

10. The use according to claim 1, **characterized in that** the pain is selected from the group consisting of traumatic pain, inflammatory pain, ischemic pain, pain caused by metabolic diseases, neuropathic pain, pain caused by tissue and organ malformation, labor pain and pain caused by malignant proliferative diseases; the traumatic pain is selected from the group consisting of pain caused by surgery, fracture pain, burn pain, abdominal traumatic pain, spinal traumatic pain, chest traumatic pain and post-traumatic headache; the inflammatory pain is selected from the group consisting of inflammatory headache, tissue inflammatory pain, organ and gland inflammatory pain and vascular inflammatory pain; the ischemic pain is selected from the group consisting of ischemic headache, limb ischemic pain, tissue ischemic pain, and organ and gland ischemic pain; the pain caused by metabolic diseases is selected from the group consisting of pain caused by gout and pain caused by diabetes; the neuropathic pain is selected from the group consisting of phantom limb pain, stump pain, burning neuralgia, postherpetic neuralgia, sympathetic-related pain, pain caused by burning foot syndrome, folic acid deficiency peripheral neuralgia, vitamin B12 deficiency peripheral neuralgia, vitamin B1 deficiency multiple neuralgia and leprosy neuralgia; the pain caused by malignant proliferative diseases is pain caused by tumors, preferably pain caused by leukemia, lymphoma, myeloma, breast cancer, lung cancer, esophageal cancer, stomach cancer, colorectal cancer, liver cancer, pancreatic cancer, head and neck cancer, kidney cancer, bladder cancer, prostate cancer, ovarian cancer, endometrial cancer, cervical cancer, osteosarcoma, soft tissue sarcoma, melanoma or brain tumor.

11. The use according to claim 10, **characterized in that** the pain is a pain with a score of 4 to 10 by the VAS pain scale, preferably selected from the group consisting of traumatic pain, labor pain, pain caused by tumors and inflammatory pain.

12. The use according to claim 11, **characterized in that** the pain with a score of 4 to 10 by the VAS pain scale is not applicable and/or not sensitive to non-opioid analgesics or weak opioid analgesics.

13. The use according to claim 1, **characterized in that** the combination also comprises a third component selected from the group consisting of an opioid, glucocorticoid, non-steroidal anti-inflammatory drug, local anesthetic, anti-depressant, calcium ion antagonist, anti-convulsant, adrenal beta blocker, anesthetic and anesthesia inducer.

14. A pharmaceutical composition, comprising the MOR agonist and the KOR agonist according to any one of claims 1 to 8, and one or more pharmaceutically acceptable excipients, diluents or carriers.
